(19) Europäisches Patentamt
European Patent Office
Office européen des brevets

(11) EP 0 624 595 B1

(12) # EUROPÄISCHE PATENTSCHRIFT

(45) Veröffentlichungstag und Bekanntmachung des Hinweises auf die Patenterteilung:
**12.08.1998 Patentblatt 1998/33**

(21) Anmeldenummer: **94106846.2**

(22) Anmeldetag: **02.05.1994**

(51) Int Cl.[6]: **C07J 9/00**, C07J 41/00, A61K 31/575, C07J 7/00 // C07J31/00, C07J51/00, C07J3/00

(54) **nor-Gallensäurederivate, Verfahren zu ihrer Herstellung und Verwendung dieser Verbindungen als Arzneimittel**

Nor-derivation of bile-acids, a process for their production and their use as medicines

Dérivés-nor-des acides biliaires, un procédé pour leur préparation et leur utilisation comme médicaments

(84) Benannte Vertragsstaaten:
**AT BE CH DE DK ES FR GB GR IE IT LI LU NL PT SE**

(30) Priorität: **08.05.1993 DE 4315370**

(43) Veröffentlichungstag der Anmeldung:
**17.11.1994 Patentblatt 1994/46**

(73) Patentinhaber: **HOECHST AKTIENGESELLSCHAFT**
**65929 Frankfurt am Main (DE)**

(72) Erfinder:
- **Enhsen, Alfons, Dr.**
  **D-64572 Büttelborn (DE)**
- **Glombik, Heiner, Dr.**
  **D-65719 Hofheim am Taunus (DE)**
- **Kramer, Werner, Dr. Dr.**
  **D-55130 Mainz (DE)**
- **Wess, Günther, Dr.**
  **D-.63526 Erlensee (DE)**

(56) Entgegenhaltungen:
**EP-A- 0 489 423** **US-A- 4 104 285**

- **CHEMICAL ABSTRACTS, vol. 113, no. 19, 5. November 1990, Columbus, Ohio, US; abstract no. 172419, HEUI SUK HAM ET AL Seite 743 ;Spalte 2 ;**
- **CHEMICAL ABSTRACTS, vol. 116, no. 11, 16. März 1992, Columbus, Ohio, US; abstract no. 103302, J. LILLIENAU ET AL 'Hepatic and Ileal Transport and Effect on Biliary Secretion of nor-Ursocholic Acid and its Conjugates in Rats' Seite 481 ;Spalte 2 ;**
- **CHEMICAL ABSTRACTS, vol. 103, no. 17, 28. Okfober 1985, Columbus, Ohio, US; abstract no. 134429, F. NAKATOMI ET AL 'Intestinal Absorption and Metabolism of Norcholic Acid in Rats' Seite 19 ;Spalte 1 ;**
- **JOURNAL OF MEDICINAL CHEMISTRY, Bd.37, Nr.7, 1. April 1994, WASHINGTON US Seiten 873 - 875 G. WESS ET AL 'Specific Inhibitors of Ileal Bile Acid Transport'**

Bemerkungen:
Die Akte enthält technische Angaben, die nach dem Eingang der Anmeldung eingereicht wurden und die nicht in dieser Patentschrift enthalten sind.

EP 0 624 595 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft Gallensäurederivate der Formel I

G1-X-G2 I,

Verfahren zu ihrer Herstellung, pharmazeutische Präparate auf Basis diese Verbindungen sowie die Verwendung der Gallensäurederivate als Arzneimittel.

Gallensäuren haben eine wichtige physiologische Funktion bei der Fettverdauung, z. B. als Cofaktoren der pankreatischen Lipasen und als natürliche Detergentien zur Solubilisierung von Fetten und fettlöslichen Vitaminen. Als Endprodukt des Cholesterinstoffwechsels werden sie in der Leber synthetisiert, in der Gallenblase gespeichert und aus dieser durch Kontraktion in den Dünndarm abgegeben, wo sie ihre physiologische Wirkung entfalten. Der größte Teil der sezernierten Gallensäuren wird über den enterohepatischen Kreislauf zurückgewonnen. Sie gelangen über die Mesenterialvenen des Dünndarms und das Pfortadersystem wieder zur Leber zurück. Bei der Rückresorption im Darm spielen sowohl aktive als auch passive Transportprozesse eine Rolle. Die Hauptmenge der Gallensäuren wird am Ende des Dünndarms, dem terminalen Ileum, durch ein spezifisches $Na^+$-abhängiges Transportsystem rückresorbiert und gelangt mit dem Mesenterialvenenblut über die Pfortader zurück zur Leber, um von den Leberzellen erneut in die Galle sezerniert zu werden. Im enterohepatischen Kreislauf treten die Gallensäuren sowohl als freie Säuren als auch in Form von Glycin- und Taurinkonjugaten in Erscheinung.

Die meisten natürlichen Gallensäuren besitzen in der Seitenkette am D-Ring des Steroidgerüstes eine C-24 Carboxylgruppe in freier oder konjugierter Form. Gallensäuren, bei denen die Seitenkette um ein oder mehrere C-Atome verkürzt ist, auch nor-Gallensäuren genannt, besitzen eine freie oder konjugierte Carboxylgruppe entsprechend an den C-Atomen 23, 22 oder 20. Manche dieser Verbindungen kommen in Spuren in natürlicher Gallenflüssigkeit vor. Nor-Gallensäuren unterscheiden sich in einigen metabolischen und physiologischen Eigenschaften von $C_{24}$-Gallensäuren (J. Lip. Res. 29, 1387, 1988).

Nichtresorbierbare, unlösliche, basische, vernetzte Polymere werden seit geraumer Zeit zur Bindung von Gallensäuren verwendet und aufgrund dieser Eigenschaften therapeutisch genutzt. In der Patentanmeldung EP-A-0 489 423 beschriebene Gallensäurederivate besitzen eine hohe Affinität zum intestinalen Gallensäuretransportsystem und erlauben daher eine spezifische Hemmung des enterohepatischen Kreislaufs. Als Therapieobjekt werden alle Erkrankungen, bei denen eine Hemmung der Gallensäureresorption im Darm, insbesondere im Dünndarm, wünschenswert erscheint, angesehen. Beispielsweise werden die cholagene Diarrhoe nach Ileumresektion, oder auch erhöhte Cholesterin-Blutspiegel auf diese Weise behandelt. Im Falle des erhöhten Cholesterin-Blutspiegels kann durch den Eingriff in den enterohepatischen Kreislauf eine Senkung dieses Spiegels erreicht werden. Durch Senkung des im enterohepatischen Kreislauf befindlichen Gallensäurepools wird die entsprechende Neusynthese von Gallensäuren aus Cholesterin in der Leber erzwungen. Zur Deckung des Cholesterinbedarfs in der Leber wird auf das im Blutkreislauf befindliche LDL-Cholesterin zurückgegriffen, wobei die hepatischen LDL-Rezeptoren vermehrt zum Einsatz kommen. Die so erfolgte Beschleunigung des LDL-Katabolismus wirkt sich durch Herabsetzung des atherogenen Cholesterinanteils im Blut aus.

Es bestand die Aufgabe, neue Arzneimittel zu finden, die in der Lage sind, den atherogenen Cholesterinanteil im Blut herabzusetzen bzw. den enterohepatischen Kreislauf hinsichtlich erhöhter Gallensäureausscheidung und daraus folgender Senkung des Cholesterinspiegels zu beeinflussen.

Diese Aufgabe wird durch die erfindungsgemäßen Gallensäurederivate abgelöst.

Die Erfindung betrifft daher Gallensäurederivate der Formel I

G1-X-G2 I

in der G1 über die Seitenkette am Ring D mit dem Verbindungsglied X an Ring A von G2 verknüpft ist, und G1 einen Rest der Formel II

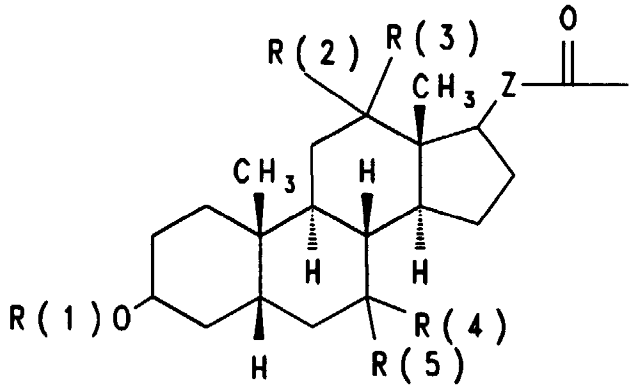

II

wobei

Z einen der folgenden Reste

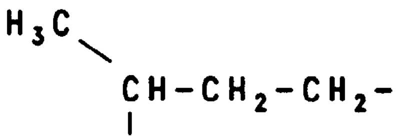 , 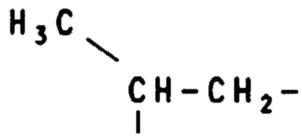 , 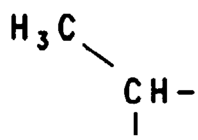 ,

oder eine einfache Bindung darstellt,

R(1) H, einen Alkylrest mit 1 bis 10 C-Atomen, einen Alkenylrest mit 2 bis 10 C-Atomen bedeutet,

R(2) bis R(5), wobei R(2) and R(3) bzw. R(4) and R(5) jeweils gemeinsam der Sauerstoff einer Carbonylgruppe oder jeweils unabhängig voneinander H oder OH bedeuten,

X eine Einfachbindung oder ein Brückenglied der Formel III ist,

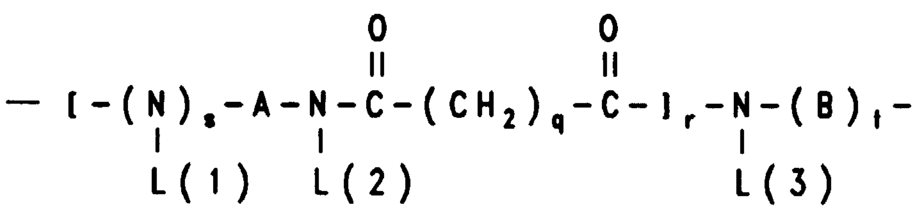

(III)

wobei

A eine Alkylenkette, die verzweigt oder unverzweigt ist und die Kette ggf. durch -O- oder -S- unterbrochen sein kann und 2 bis 6 Kettenglieder p umfaßt,

B eine Alkylenkette, die verzweigt oder unverzweigt ist und die Kette ggf. durch -O- oder -S- unterbrochen sein kann und die Kette 2 bis 6 Kettenglieder n umfaßt,

L(1), L(2), L(3) gleich oder verschieden sind und H, $C_1$-$C_4$-Alkyl oder Benzyl bedeuten, und

q 0 bis 5
r 0 oder 1
s 0 oder 1

t 0 oder 1

bedeuten, und

G2 einen Rest der Formel IV bedeutet

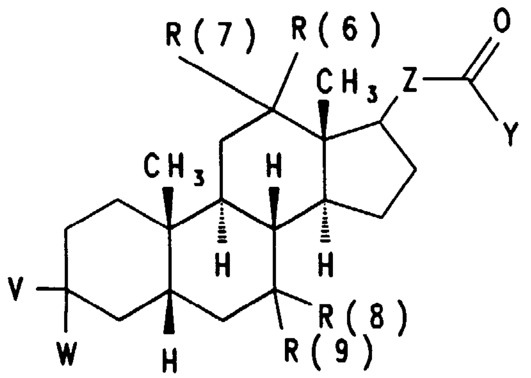

IV

wobei

Z einen der folgenden Reste

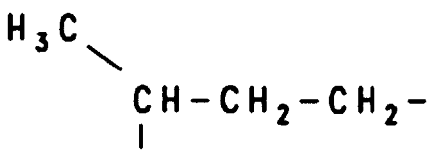 , 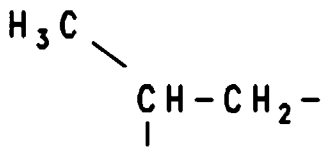 , 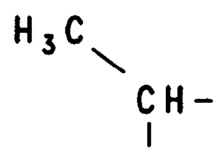 ,

oder eine einfach Bindung bedeutet,

V

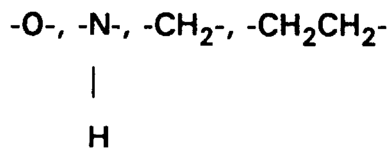

W H oder, falls V = $-CH_2-$, $-CH_2CH_2-$ ist, kann W H oder OH sein,

Y -OL, -NHL,

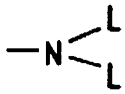

eine über die Aminogruppe gebundene Aminosäure oder Aminosulfonsäure, ausgewählt aus der Gruppe bestehend aus $-NH-CH_2-COOH$, $-NH-CH_2-CH_2-SO_3H$,

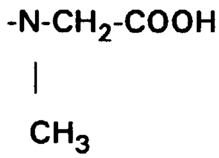 und 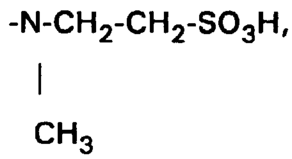,

wobei L

H, einen Alkylrest mit bis zu 10 C-Atomen, einen Alkenylrest mit 2 bis 10 C-Atomen, einen Cycloalkylrest mit 3 bis 8 C-Atomen, einen Phenylrest, der unsubstituiert ist oder 1- bis 3-fach substituiert ist mit F, Cl, Br, $(C_1-C_4)$-Alkyl oder $(C_1-C_4)$-Alkoxy, einen Benzylrest, der unsubstituiert oder 1 bis 3fach substituiert ist mit F, Cl, Br, $(C_1-C_4)$-Alkyl oder $(C_1-C_4)$-Alkoxy bedeutet, und

R(6) - R(9), wobei R(6) und R(7) bzw. R(8) und R(9) jeweils gemeinsam der Sauerstoff einer Carbonylgruppe oder einzeln und jeweils unabhängig voneinander H, -OH sind, und mindestens einer der beiden Gallensäurereste G1 oder G2 in der Seitenkette um ein oder mehrere C-Atome verkürzt ist, d.h. Z eine Bindung,

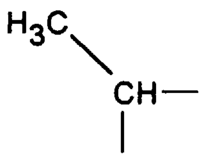 oder 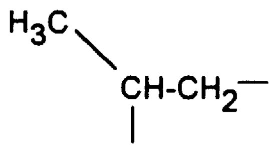

bedeutet.

Sofern nicht anders angegeben, sind Alkyl-, Alkenyl- und Alkoxygruppen geradkettig oder verzweigt.

Die erfindungsgemäßen Verbindungensind herstellbar, indem man

a) im Falle von X = Einfachbindung geeignete reaktionsfähige Formen von G1 und G2 nach im Prinzip bekannten Verfahren miteinander zur Reaktion bringt oder

b) im Falle von X = Brückengruppe

α) reaktionsfähige Formen von G1-X mit G2 bzw.
β) reaktionsfähige Formen von G2-X mit G1

nach im Prinzip bekannten Verfahren miteinander zur Reaktion bringt.

Die Verfahren können wie folgt durchgeführt werden:

a) X = Einfachbindung

Die Gallensäuren G1 werden entweder in freier Form oder in geschützter Form eingesetzt. Nach der Verknüpfung mit G2, die ebenfalls in freier oder geschützter Form vorliegt, erfolgt gegebenenfalls die Abspaltung der Schutzgruppen und die Umwandlung der Carboxylfunktion in ein obengenanntes Derivat. Als Schutzgruppen für die Alkoholgruppen eignen sich zweckmäßigerweise Formyl, Acetyl, Tetrahydropyranyl oder t-Butyldimethylsilyl. Als Schutzgruppen für die Carboxylgruppe der Seitenkette kommen verschiedene Alkyl- oder Benzylester in Frage, aber auch z. B. Orthoester. Beispielsweise reagiert Gallensäure bevorzugt an Position 3, aber auch an Position 7 mit aktivierten Formen von Carbonsäuren, wie Säurechloriden oder gemischten Anhydriden unter Zusatz von Basen wie Trialkylamin, Pyridin, aber auch NaOH bei Zimmertemperatur in geeigneten Lösungsmitteln wie Tetrahydrofuran, Methylenchlorid oder Essigester, aber auch Dimethylformamid (DMF) oder Dimethoxyethan (DME).

Die verschiedenen Isomeren können z. B. chromatographisch getrennt werden. Durch die Verwendung von geeigneten Schutzgruppen läßt sich die Reaktion selektiv durchführen.

Analog lassen sich entsprechende Aminogallensäuren in entsprechende Amide überführen. Auch hier kann die Reaktion entweder mit geschützten oder freien Gallensäuren durchgeführt werden.

Analog lassen sich weitere erfindungsgemäße Verbindungen nach bekannten Standardverfahren verknüpfen.

b) X = eine Brückengruppe

Die unter a) angegebenen Verfahren werden auch angewendet, um die Verknüpfung G1-X mit G2 bzw. G1 mit X-G2 durchzuführen.

Zweckmäßigerweise setzt man auch hier den Gallensäureteil entweder geschützt oder ungeschützt ein.

Die Herstellung von reaktionsfähigen Gallensäurebausteinen ist in den folgenden Formelschemata (1 bis 4) am Beispiel von Cholsäurederivaten beschrieben (Schemata 1 und 2 betreffen die Verkürzung der Seitenkette, Schemata 3 und 4 betreffen die Aktivierung in 3-Stellung).

## Schema 1:

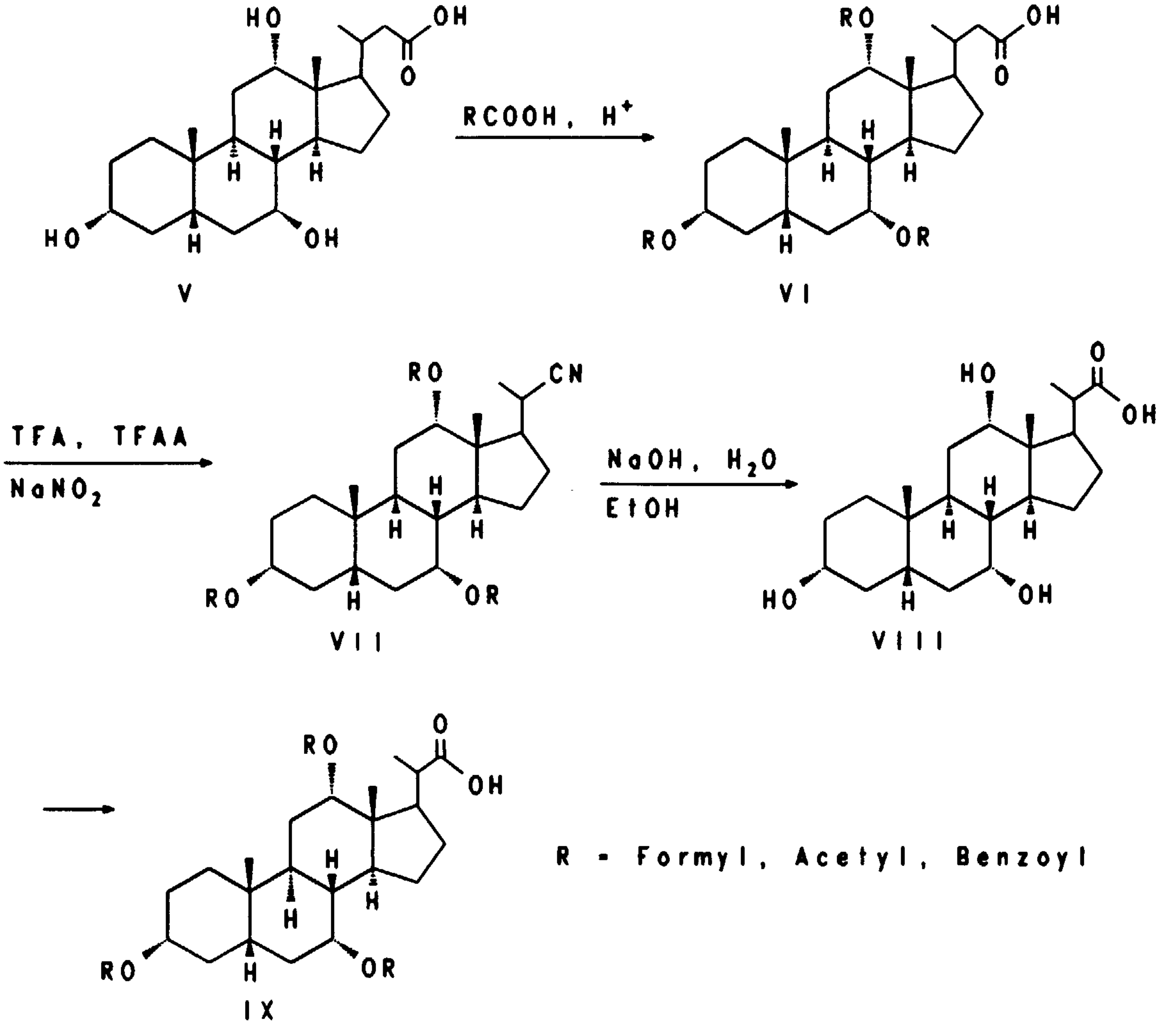


V, VI, VII, VIII, IX

R = Formyl, Acetyl, Benzoyl

Die Synthese von nor-Cholsäure V aus Cholsäure wurde von Hofmann et al. (J. Lip. Res. 29, (1988), 1287) beschrieben. Nach diesem Verfahren kann die Seitenkette auch weiter zur C-22 Carbonsäure VIII bzw. IX verkürzt werden. Die Hydroxygruppen der nor-Gallensäure V werden geschützt z. B. mit Formyl-, Acetyl- oder Benzoylschutzgruppen (R). Durch Umsetzung der Verbindung VI mit Trifluoressigsäure/Trifluoressigsäureanhydrid/Natriumnitrit erhält man das Nitril VII, das durch Verseifung mit starker Base zur freien, um ein weiteres C-Atom verkürzte Gallensäure VIII führt. Zur weiteren Umsetzung kann diese Verbindung ungeschützt oder in erneut geschützter Form IX (wobei als R auch Acyl-, Silyl- oder auch Tetrahydropyranyl(THP)-Schutzgruppen in Betracht kommen) eingesetzt werden.

## Schema 2:

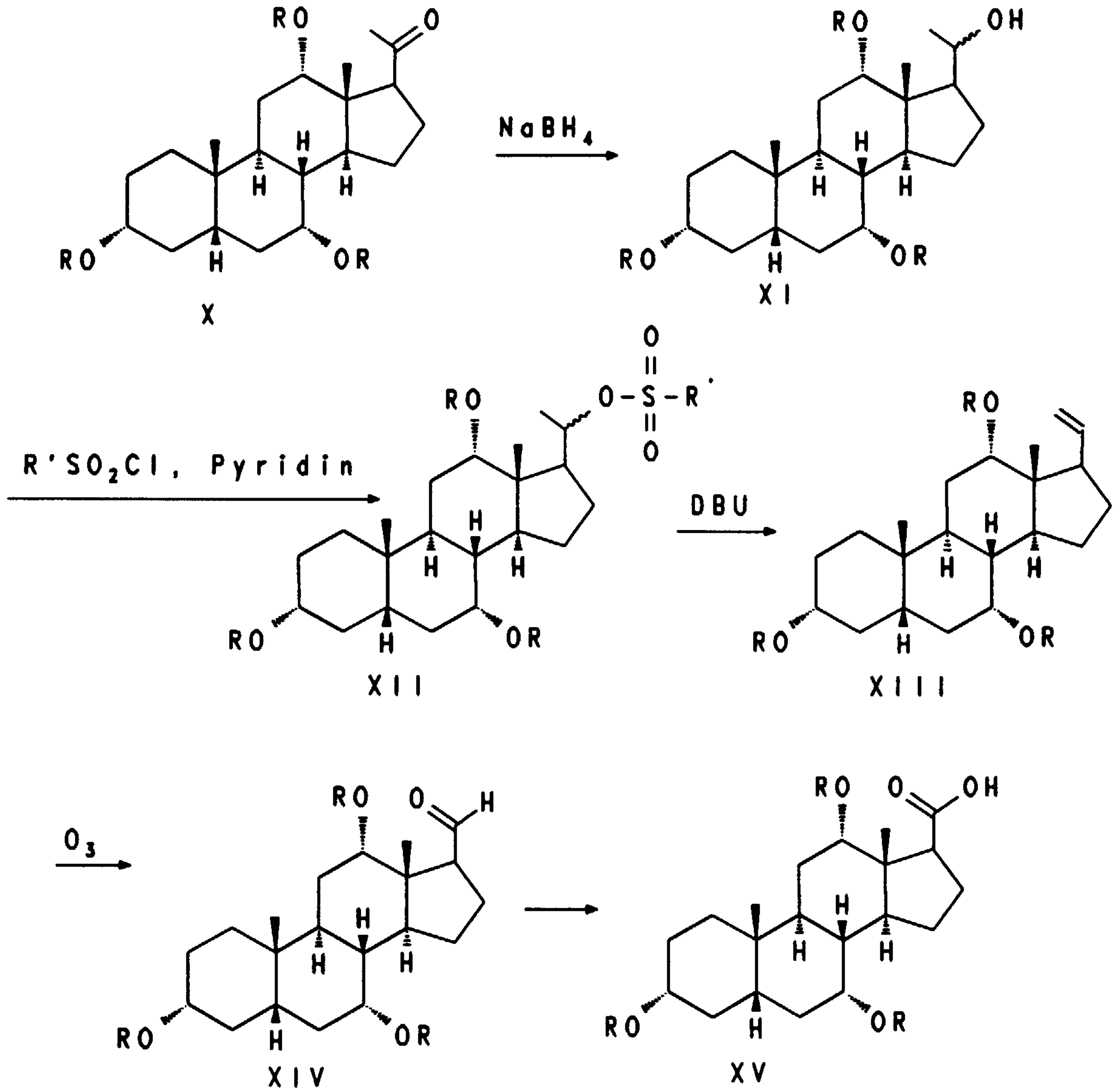


X XI XII XIII XIV XV

R = Formyl, Acetyl        R' = $CH_3$, $CF_3$

Die Verbindung X wird mit Natriumborhydrid zum Alkohol XI reduziert. Die Alkoholfunktion wird aktiviert, zum Beispiel durch einen Methansulfonyl- oder Trifluormethansulfonylrest wie in Verbindung XII, und anschließend mit einer geeigneten Base, z. B. Diazabicycloundecen, zum vinylischen System XIII eliminiert. Die 20,21-Doppelbindung wird mit Osmiumtetroxid/Natriumperjodat oder Ozon in einem geeigneten Lösungsmittel zum Aldehyd XIV gespalten. Oxidation von XIV nach bekannten Verfahren, z. B. mit Natriumchlorit, führt zum entsprechenden C-20-Carboxyl-Gallensäurederivat XV.

## Schema 3:

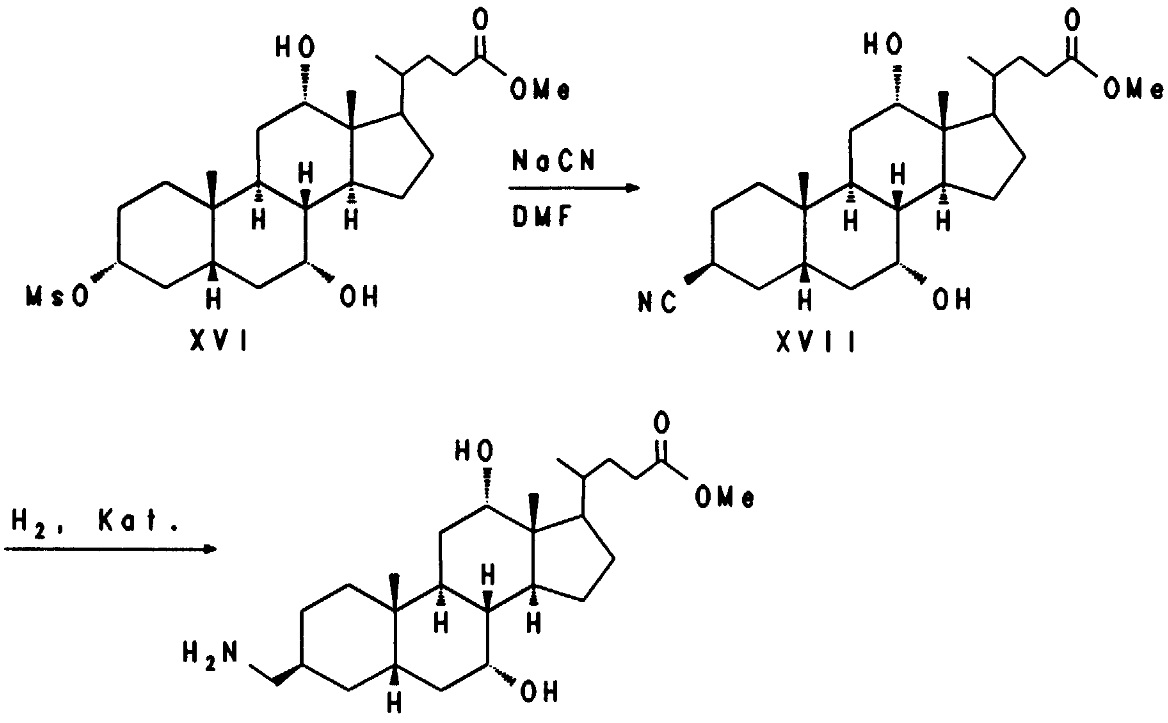


XVI XVII

XVIII

Nach Aktivierung der 3-Hydroxyfunktion, z. B. durch eine Methansulfonylgruppe wie bei Verbindung XVI, kann durch nukleophile Substitution mit Natriumcyanid in einem geeigneten Lösungsmittel bei erhöhter Temperatur eine Nitrilgruppe eingeführt werden. Die so erhaltene Verbindung XVII kann durch Hydrierung mit Wasserstoff in Gegenwart eines geeigneten Katalysators z. B. Rhodium/$Al_2O_3$ in den Aminomethylbaustein XVIII überführt werden.

**Schema 4:**

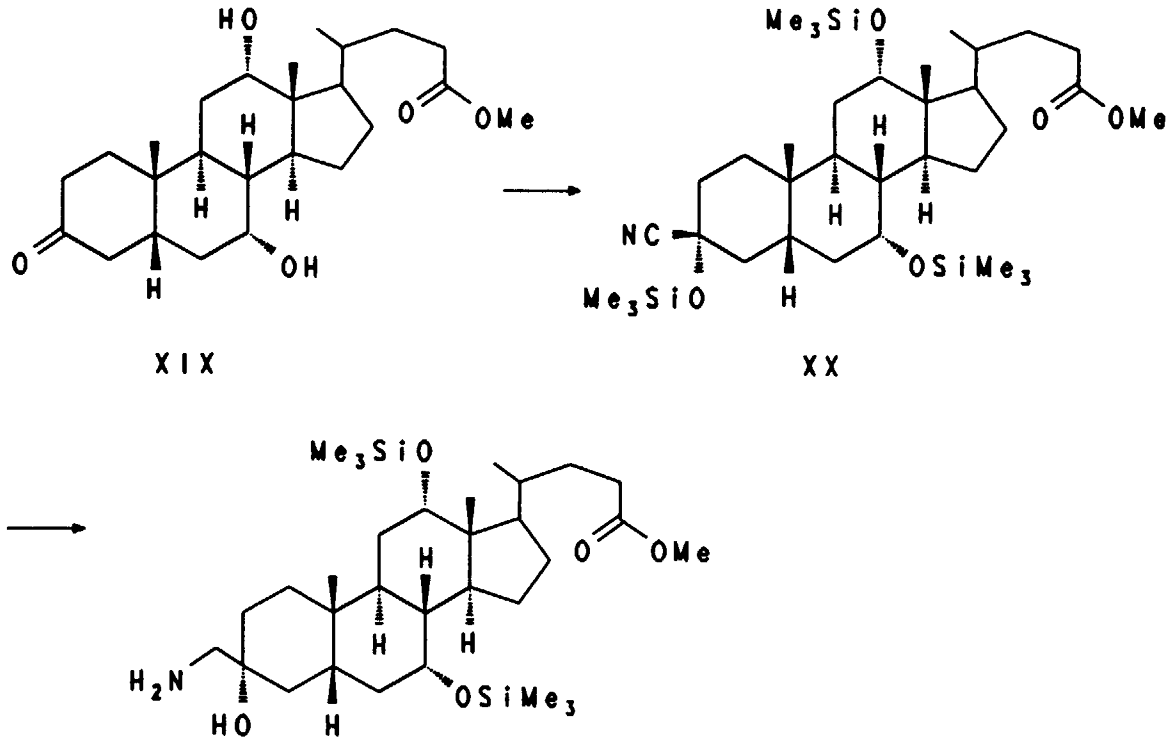

XIX

XX

XXI

Trimethylsilylcyanid kann in guten Ausbeuten an 3-Ketocholansäureester XIX addiert werden. Unter den gewählten Reaktionsbedingungen (TMSCN, $ZnJ_2$, $CH_2Cl_2$) erhält man ein Nitrilderivat XX, in dem alle Hydroxygruppen silyliert sind. Durch den großen Raumbedarf der TMS-Gruppe wird die Nitrilgruppe in die axiale β-Position dirigiert, die Diastereoselektivität ist > 9:1 (Lit.: Evans et al., J. Org. Chem. 39 (1974) 914). Die Nitrilfunktion läßt sich mit Natriumborhydrid/Trifluoressigsäure in THF zum 3β-Aminomethylderivat XXI reduzieren.
Auf diesem Weg erhält man Cholansäurederivate, bei denen neben einem zusätzlichen Linker (X) die ursprüngliche 3α-Hydroxyfunktion erhalten bleibt.

Die Erfindung betrifft weiterhin die Verwendung der erfindungsgemäßen Verbindungen zur Herstellung eines Heilmittels.
Hierzu werden die Verbindungen der allgemeinen Formel I gelöst oder suspendiert in pharmakologisch unbedenklichen organischen Lösungsmitteln, wie ein- oder mehrwertigen Alkoholen, wie z. B. Ethanol oder Glycerin, in Triacetin, Ölen wie z. B. Sonnenblumenöl, Lebertran, Ethern, wie z. B. Diethylenglykoldimethylether oder auch Polyethern wie z. B. Polyethylenglykol, oder auch in Gegenwart anderer pharmakologisch unbedenklicher Polymerträger, wie z. B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen in Kombination mit anderen Arzneistoffe, gegeben werden.
Die Verbindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 5000 mg, vorzugsweise jedoch in Dosisbereich 10 bis 1000 mg.

Die Verbindungen eignen sich aufgrund ihrer pharmakologische Eigenschaften insbesondere als Hypolipidämika.

Die Erfindung betrifft daher auch Arzneimittel auf Basis der Verbindungen der Formel (I) sowie die Verwendung der Verbindungen als Arzneimittel, insbesondere zur Senkung des Cholesterinspiegels.

Die biologische Prüfung der erfindungsgemäßen Verbindungen erfolgte durch Ermittlung der Hemmung der [$^3$H]-Taurocholataufnahme in Bürstensaummembranvesikel des Ileums von Kaninchen. Der Hemmtest wurde wie folgt durchgeführt:

1. Präparation von Bürstensaummembranvesikeln aus dem Ileum von Kaninchen

Die Präparation von Bürstensaummembranvisikeln aus den Darmzellen des Dünndarm erfolgte mit der sogenannten $Mg^{2+}$-Präzipitationsmethode. Männliche Neuseeland-Kaninchen (2 bis 2,5 kg Körpergewicht) wurden durch intravenöse Injektion von 0,5 ml einer wäßrigen Lösung von 2,5 mg Tetracain HCl, 100 T 61$^{R}$ und 25 mg Mebezoniumjodid getötet. Der Dünndarm wurde entnommen und mit eiskalter physiologischer Kochsalzlösung gespült. Die terminalen 7/10 des Dünndarms (gemessen in oral rectaler Richtung, d. h. das terminale Ileum, welches das aktive $Na^{+}$-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethylsulfonylfluorid/1 mg/l Trypsin Inhibitor v. Sojabohnen (32 U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, BRD) 3 Minuten bei 75 % max. Leistung unter Eiskühlung homogenisiert. Nach Zugabe von 3 ml 1 M $MgCl_2$-Lösung (Endkonzentration 10 mM) ließ man exakt 1 Minute bei 0°C stehen. Durch Zugabe von $Mg^{2+}$ aggregieren die Zellmembranen und präzipitieren mit Ausnahme der Bürstensaummembranen. Nach einer 15-minütigen Zentrifugation bei 3000 x g (5000 rpm, SS-34-Rotor) wurde der Niederschlag verworfen und der Überstand, der die Bürstensaummembranen enthielt, 30 Minuten bei 267000 x g (15000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-minütiger Inkubationszeit bei 0°C wurde erneut 15 Minuten bei 3000 x g zentrifugiert. Der Überstand wurde anschließend nochmals 30 Minuten bei 46000 x g (15000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

2. Hemmung der $Na^{+}$-abhängigen [$^{3}H$]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [$^{3}H(G)$]-Taurocholat (spezifische Aktivität: 2,1 Ci/mMol) /0,5 µl 10 mM Taurocholat/8,75 µl Natrium-Transport-Puffer (10 mM Tris/Hepes, (pH 7,4)/100 mM Mannit/100 mM NaCl) (Na-T-P) bzw. 8,75 µl Kalium-Transport-Puffer (10 mM Tris/Hepes (pH 7,4)/ 100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm Ø, Millipore, Eschborn, BRD) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, BRD) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, BRD) aufgelöst und die Radioaktivität durch Flüssigkeits-Szintillationsmessung in einem Meßgerät TriCarb 2500 (Canberra Packard GmbH, Frankfurt, BRD) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den $Na^{+}$-abhängigen Transportanteil. Als $IC_{50}$ $Na^{+}$ wurde diejenige Konzentration an Inhibitor bezeichnet, bei der der $Na^{+}$-abhängige Transportanteil um 50 % - bezogen auf die Kontrolle - gehemmt war.

Die pharmakologischen Daten umfassen eine Testserie, in der die Interaktion der erfindungsgemäßen Verbindungen mit dem intestinalen Gallensäuretransportsystem im terminalen Dünndarm untersucht wurde. Die Ergebnisse sind in Tabelle 11 zusammengefaßt.

Beispiel 1

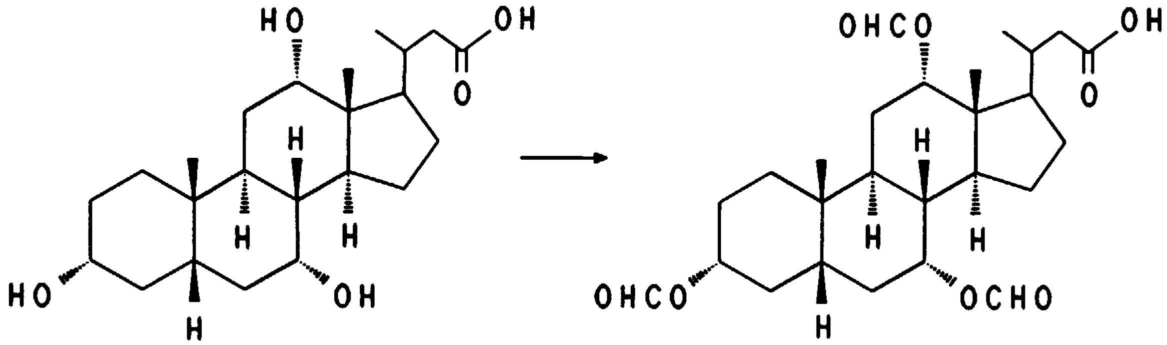

48 g (122 mmol) 3α,7α,12α-Trihydroxy-24-nor-23-cholansäure (=Norcholsäure), 200 ml Ameisensäure und 1 ml Perchlorsäure (60 %) werden 1,5 Stunden bei 50°C gerührt, auf Zimmertemperatur abgekühlt, mit 160 ml Essigsäureanhydrid versetzt und noch 15 Minuten gerührt. Das Gemisch wird auf 1,5 l Wasser gegossen, die festen Bestandteile werden abgesaugt und mit 1 l Wasser gewaschen. Der Rückstand wird in 700 ml Ether gelöst und dreimal mit Wasser gewaschen. Die organische Phase wird getrocknet ($MgSO_4$) und eingeengt. Ausbeute 52 g (89 %) Beispiel 1.
MS (FAB, 3-NBA/LiCl) $C_{26}H_{38}O_8$ (478), 485 ($M+Li^+$)

Beispiel 2

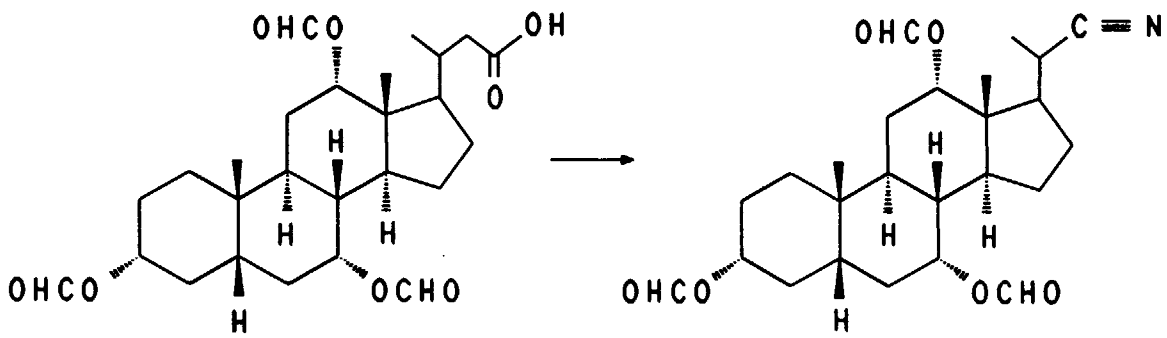

5 g (10,4 mmol) Beispiel 1 werden bei 0°C in 20 ml Trifluoressigsäure/5 ml Trifluoressigsäureanhydrid gelöst. Innerhalb einer Stunde werden portionsweise 840 mg (12 mmol) Natriumnitrit zugegeben. Es wird noch eine Stunde bei 0°C, dann 2 Stunden bei 40°C nachgerührt. Die Lösung wird wieder auf 0°C gekühlt, mit 5N NaOH neutralisiert und mit Dichlormethan extrahiert. Die organische Phase wird getrocknet ($MgSO_4$) und eingeengt. Chromatographie des Rückstands über Kieselgel (Cyclohexan/Essigester = 2:1) ergibt 3,1 g (67 %) Beispiel 2.
MS (FAB, 3-NBA/LiCl) $C_{25}H_{35}NO_6$ (445), 452 ($M+Li^+$)

Beispiel 3

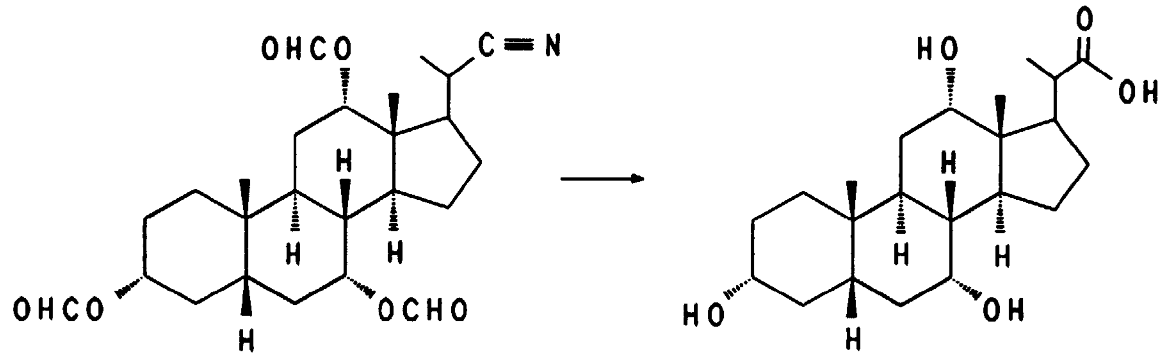

1,5 g (3,37 mmol) Beispiel 2 und 5 g KOH werden in 50 ml Ethanol/Wasser (= 1:1) gelöst und unter Rückfluß erhitzt. Nach Beendigung der Reaktion (DC-Kontrolle) wird das Ethanol abgezogen und der Rückstand mit Ether gewaschen. Die wäßrige Phase wird mit 2N HCl angesäuert und dreimal mit Essigester extrahiert. Die vereinigten organischen Phasen werden getrocknet ($MgSO_4$) und eingeengt. Man erhält 1,25 g (97 %) Beispiel 3.
MS (FAB, 3-NBA/LiCl) $C_{22}H_{36}O_5$ (380), 387 ($M+Li^+$)

Beispiel 4

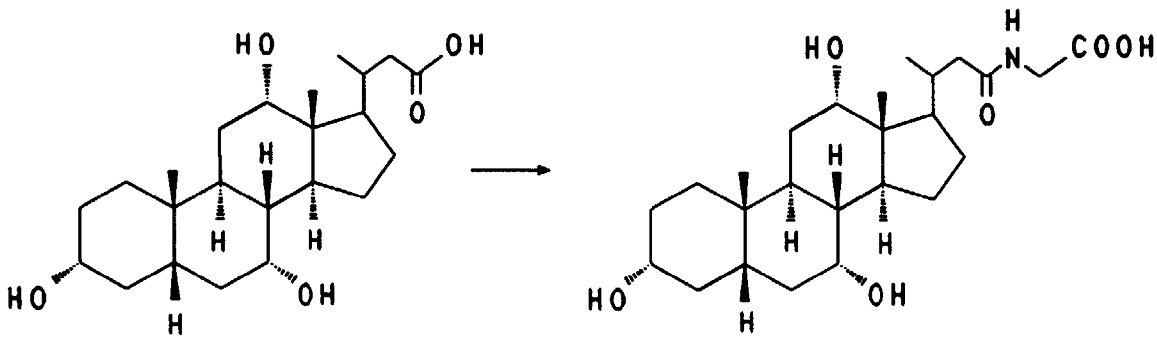

500 mg (12,87 mmol) 3α,7α,12α-Trihydroxy-24-nor-23-cholansäure und 370 mg (36 mmol) N-Methylmorpholin werden in 20 ml THF gelöst. Bei 10°C werden 0,34 ml (36 mmol) Chlorameisensäureethylester zugegeben. Nach 15 Minuten wird eine Lösung von 270 mg (36 mmol) Glycin in 5 ml 1N NaOH zugetropft. Es wird 18 Stunden bei Zimmertemperatur nachgerührt. Das Reaktionsgemisch wird eingeengt und der Rückstand wird über Kieselgel chromatographiert (Dichlormethan/Methanol = 8:2). Man erhält 320 mg (56 %) Beispiel 4.
MS (FAB/ 3-NBA) $C_{25}H_{41}NO_6$ (451), 452 ($M+H^+$)

Beispiel 5

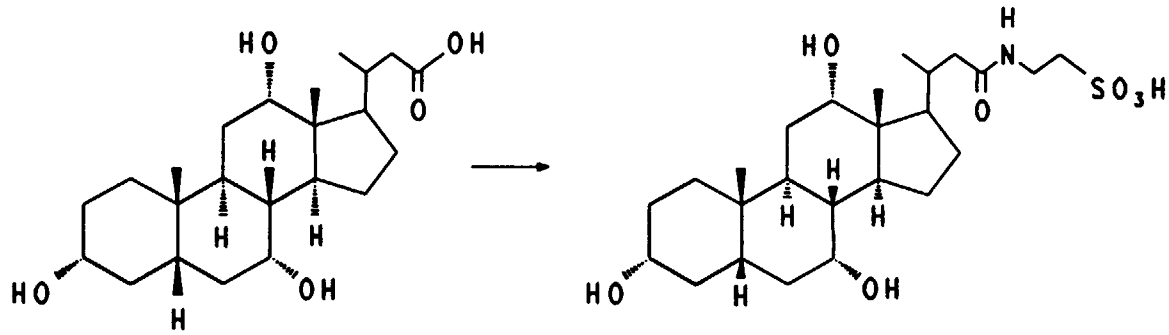

Nach dem für Beispiel 4 beschriebenen Verfahren werden aus 500 mg (12,67 mmol) Norcholsäure und 450 mg (836 mmol) Taurin 340 mg (53 %) Beispiel 5 erhalten.
MS (FAB, 3-NBA) $C_{25}H_{43}NO_7S$ (501), 502 ($M+H^+$)

Beispiel 6

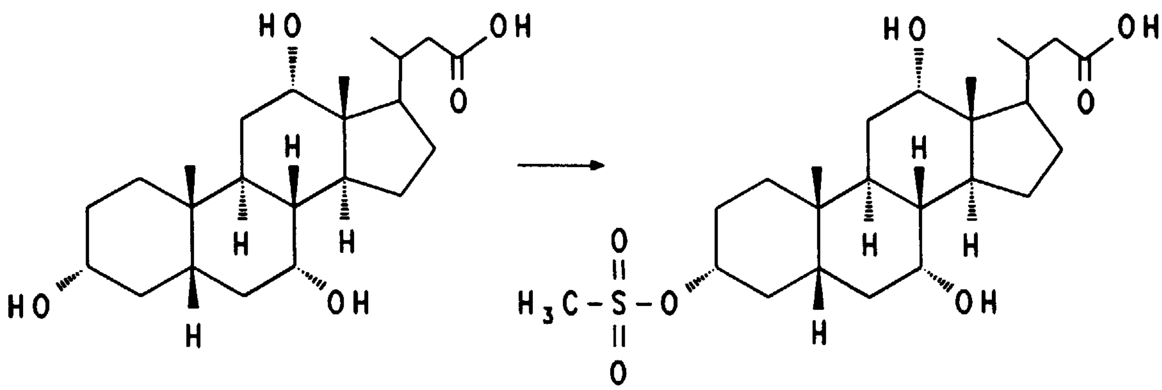

10 g (25,3 mmol) Norcholsäure werden in 50 ml Pyridin gelöst. Bei 0°C werden 2,6 ml Methansulfonsäurechlorid zugetropft. Es wird anschließend 3 Stunden bei Zimmertemperatur gerührt. Das Reaktionsgemisch wird auf Eiswasser gegossen und dreimal mit Essigester extrahiert. Die organische Phase wird getrocknet ($MgSO_4$) und eingeengt. Das Rohprodukt wird aus Diisopropylether kristallisiert, abgesaugt und anschließend im Vakuum getrocknet. Es werden 11,2 g (93 %) Beispiel 6 erhalten.
MS (FAB, 3-NBA/LiCl) $C_{24}H_{40}O_7S$ (472), 485 ($M+2Li^+-H^+$)

Beispiel 7

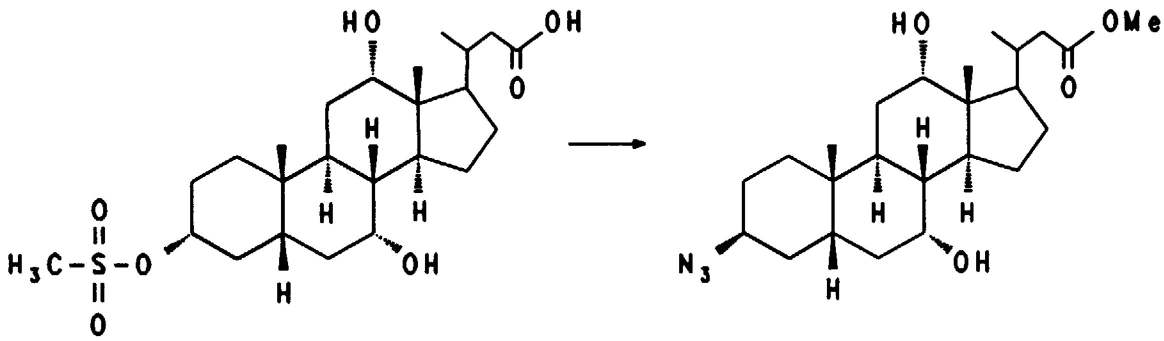

38,7 g (81,9 mmol) Beispiel 6 und 6,9 g (106 mmol) Natriumazid werden 2,5 Stunden bei 130°C in 350 ml Dimethylformamid gerührt. Nach dem Abkühlen wird auf 1,5 l Eiswasser gegossen und dreimal mit Essigester extrahiert. Die organische Phase wird getrocknet ($MgSO_4$) und eingeengt. Das Rohprodukt wird in einer aus 100 ml Methanol und 14 ml Acetylchlorid hergestellten methanolischen Salzsäurelösung 2 Stunden bei Zimmertemperatur verestert. Zur Aufarbeitung wird teilweise eingeengt, das Produkt wird auf 1 l Wasser gegossen und dreimal mit Essigester extrahiert. Nach dem Trocknen und Einengen der organischen Phase wird das Rohprodukt über Kieselgel chromatographiert (Cyclohexan/Essigester = 6:4). Es werden 9,0 g (25 %) Beispiel 7 erhalten.
MS (FAB, 3-NBA/LiCl) $C_{24}H_{39}N_3O_4$ (433), 440 ($M+Li^+$)

Beispiel 8

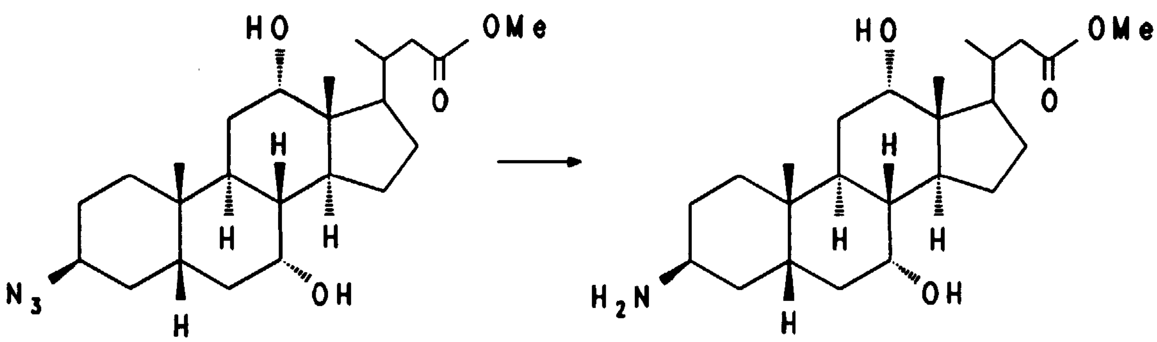

8,0 g (18,5 mmol) Beispiel 7 werden in 220 ml Essigester mit Wasserstoff in Gegenwart von ca. 50 mg 10 % Pd/C hydriert. Nach Beendigung der Reaktion wird vom Katalysator abfiltriert und das Filtrat wird eingeengt. Chromatographie des Rückstands (Methanol/Triethylamin = 95:5) ergibt 6,0 g (80 %) Beispiel 8.
MS (FAB, 3-NBA/LiCl) $C_{24}H_{41}NO_4$ (407), 414 ($M+Li^+$)

Beispiel 9

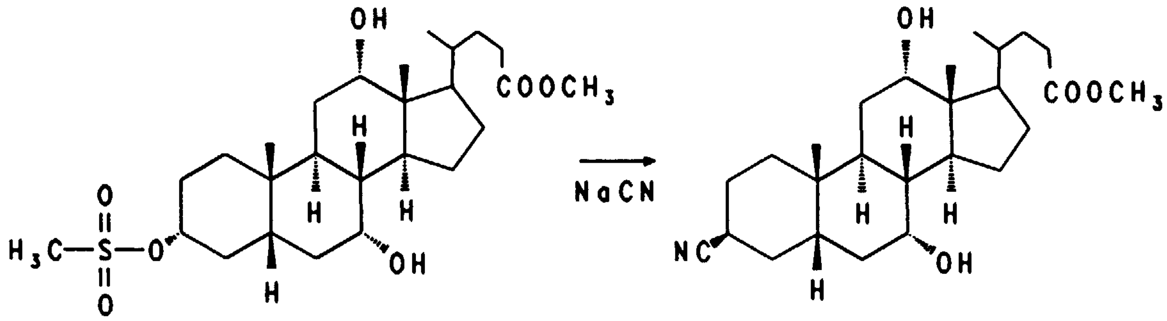


4,3 g (8,6 mmol) des Mesylats (vergl. EP-A-0 489 423) werden in 80 ml trockenem DMF mit 0,42 g (8,6 mmol) Natriumcyanid 3 Stunden auf 100 bis 110°C erhitzt. Man gießt auf Eiswasser, extrahiert mit Ethylacetat und filtriert den Rückstand der organischen Phase über Kieselgel. (Ethylacetat/Heptan = 2:1). Man erhält 890 mg (25 %) Nitril.
MS (FAB, 3-NBA/LiCl) $C_{26}H_{41}NO_4$ (431), 438 (M+Li$^+$)

Beispiel 10

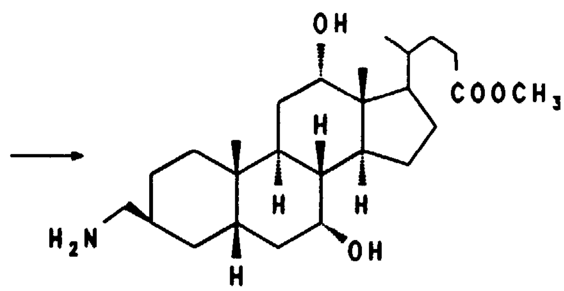

1,5 g (3,48 mmol) des Nitrils aus Beispiel 9 werden in 100 ml Methanol unter Zusatz von 10 ml konz. Ammoniaklösung und 1 g 5 %igem Rhodium auf $Al_2O_3$ 24 Stunden bei 140 bar und 50°C hydriert. Man saugt vom Katalysator ab, engt das Filtrat ein und reinigt den Rückstand über Kieselgel ($CH_2Cl_2$/MeOH/konz. $NH_3$-Lösung = 100:15:2). Man erhält 1,1 g (73 %) Amin (Beispiel 10).
MS (FAB, 3-NBA/LiCl) $C_{26}H_{45}NO_4$ (435), 442 (M+Li$^+$)

Beispiel 11A

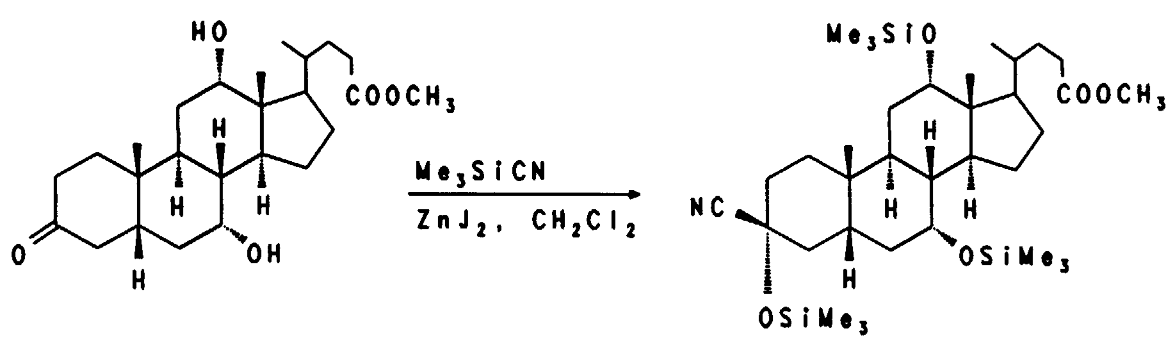


9 g (21,4 mmol) Keton (vergl. Schema 4) werden unter Argon in 50 ml trockenem Dichlormethan mit 270 mg trockenem Zinkiodid und unter Eiskühlung portionsweise mit 10 ml (3,5 Äquiv.) Trimethylsilylcyanid versetzt. Nach ca. 1,5 Stunden ist die Umsetzung beendet. Der nach dem Einengen verbleibende Rückstand wird mit n-Heptan-Ethylacetat = 10:1 über Kieselgel gereinigt. Man erhält 12,1 g (85 %) des Produkts als farbloses Öl, das ganz überwiegend (> 9:1) aus

einem Stereoisomer besteht.
MS (FAB, 3-NBA, LiCl) $C_{35}H_{65}NO_5Si_3$ (664), 671 (M+Li+)

Beispiel 11B

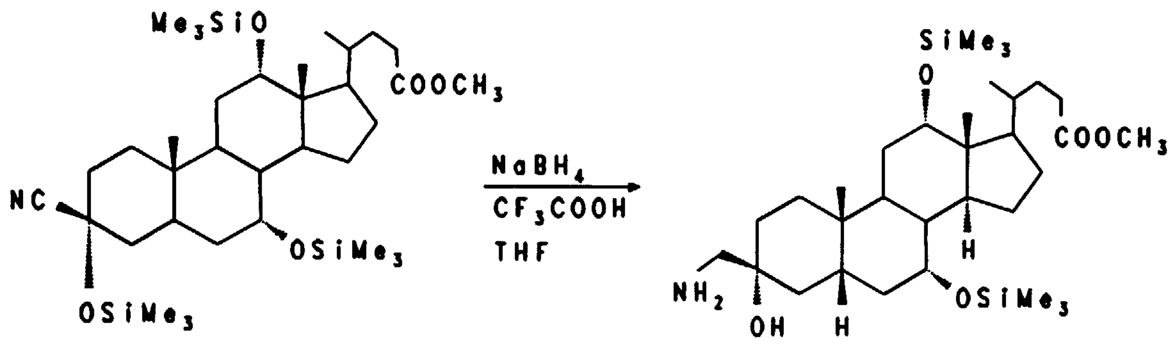


Zu einer Suspension von 1,036 g (827,4 mmol) Natriumborhydrid in trockenem THF gibt man zunächst 2,1 ml (27,4 mmol) Trifluoressigsäure, rührt 15 Minuten und fügt dann unter Eiskühlung 12,1 g (18,2 mmol) des Nitrils aus Beispiel 11A in 40 ml trockenem THF hinzu. Nach 24 Stunden bei Raumtemperatur wird durch Zusatz von Wasser und Ether aufgearbeitet, die organische Phase mit Hydrogencarbonatlösung ausgeschüttelt und der Rückstand mit $CH_2Cl_2/CH_3OH$/konz. $NH_3$-Lösung = 100:10:1,5 chromatographisch gereinigt. Man erhält 7,83 g (48 %) des Amins.
MS (FAB), 3-NBA, LiCl) $C_{32}H_{61}NO_5Si_2$ (596), 603 (M+Li+)

Beispiel 12A

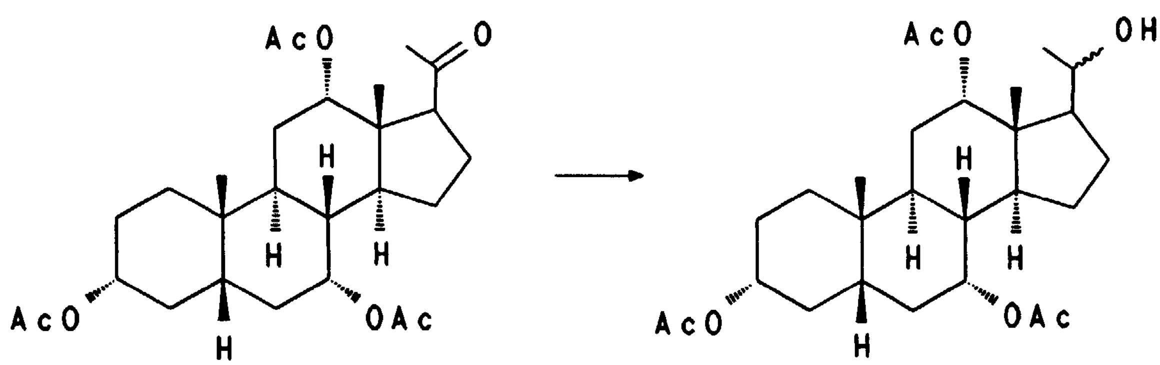

20 g (42 mmol) Methylketon (vergl. Schema 2) werden in 400 ml Methanol gelöst, mit 2,48 g (64 mmol) Natriumborhydrid versetzt und 45 Minuten bei Zimmertemperatur gerührt. Nach der Zugabe von 400 ml Wasser wird vorsichtig 2N HCl zugegeben bis pH 3 erreicht ist. Es wird eingeengt, erneut Wasser zugegeben und mit EE extrahiert. Die organische Phase wird getrocknet, eingeengt und der Rückstand wird über Kieselgel chromatographiert (Cyclohexan/Essigester 1:1).
Ausbeute: 15,1 g (75 %).
MS (FAB, 3-NBA/LiCl) $C_{27}H_{42}O_7$ (478), 485 (M+Li+)

Beispiel 12B

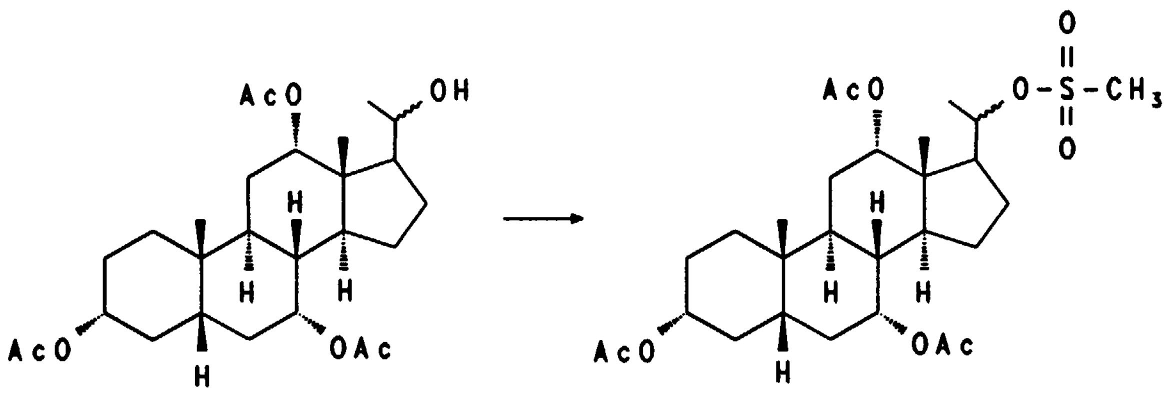

15,1 g (31,5 mol) Alkohol (Beispiel 12A) wird in 250 ml Dichlormethan/250 ml Pyridin gelöst, bei 0°C mit 4 g (35 mmol) Methansulfonsäurechlorid versetzt und 2 Stunden bei Zimmertemperatur gerührt. Zur Aufarbeitung wird Wasser zugegeben und mit Essigester extrahiert. Nach dem Trocknen und einengen der Essigesterphase werden 17,5 g (quart.) Mesylverbindung erhalten, die ohne weitere Reinigung umsetzt werden kann.
MS (FAB, 3-NBA/LiCl) $C_{28}H_{44}O_9S$ (556), 563 ($M+Li^+$)

Beispiel 12C

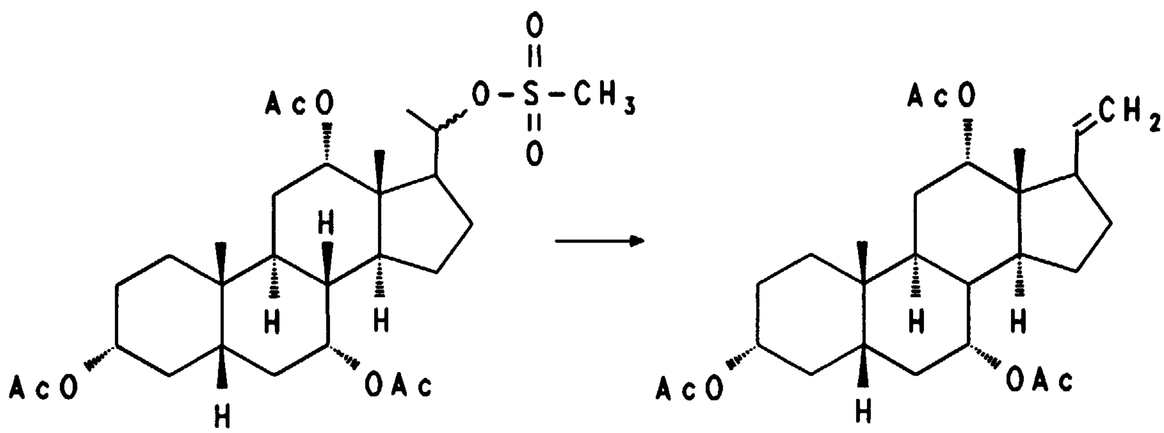

18 g (32,3 mmol) Beispiel 12B und 80 ml Diazabicycloundecen werden in 400 ml DMF gelöst. Es wird 16 Stunden bei 100°C gerührt. Nach dem Abkühlen wird das Reaktionsgemisch eingeengt und über Kieselgel chromatographiert (Cyclohexan/Essigester = 7:3). Die Ausbeute beträgt 9,6 g (64 %).
MS (FAB, 3-NBA/LiCl) $C_{27}H_{40}O_6$ (460), 467 ($M+Li^+$)

Beispiel 12D

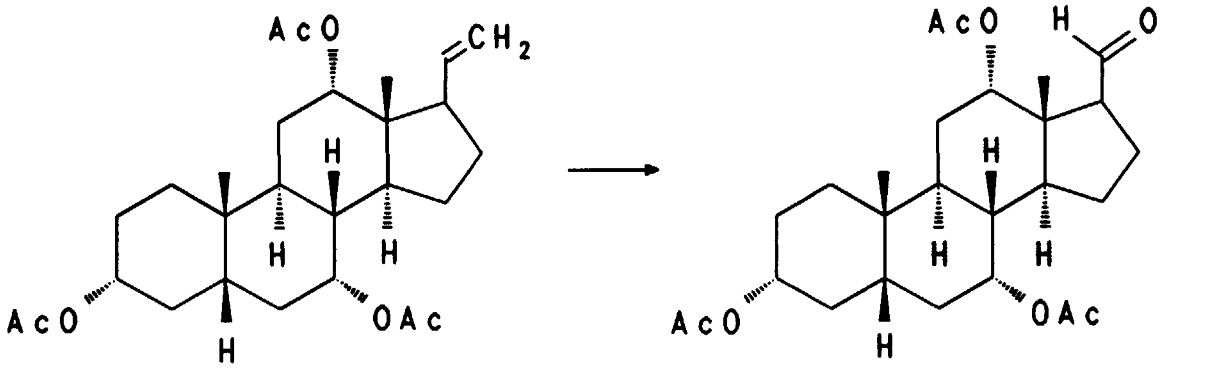

13,g (28,2 mmol) Beispiel 12C werden in 100 ml Dichlormethan gelöst, mit 10 ml Pyridin versetzt und auf -60°C gekühlt. Unter Rühren wird bis zur Blaufärbung Ozon eingeleitet. Anschließend wird mit $N_2$ gespült, auf Zimmertemperatur erwärmt und Dimethylsulfid zugegeben. Das Reaktionsgemisch wird eingeengt und der Rückstand wird über Kieselgel chromatographiert (Cyclohexan/Essigester = 7:3). Man erhält 5,8 g (44 %) Aldehyd.
MS (FAB, 3-NBA/LiCl) $C_{26}H_{38}O_7$ (462), 469 ($M+Li^+$)

Beispiel 12E

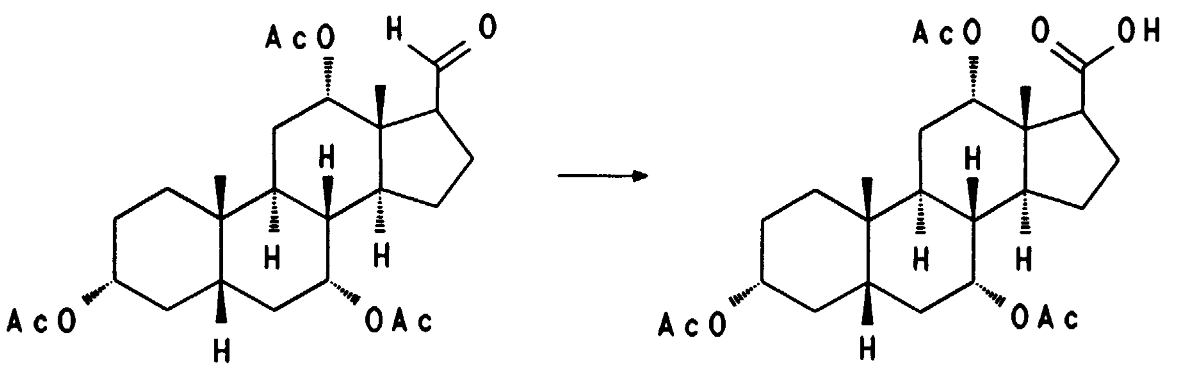

Der Aldehyd Beispiel 12D wird durch Jones Oxidation (J. Chem. Soc. 1953, 2548) zur freien C-20-Carbonsäure Beispiel 12E oxidiert.
MS (FAB, 3-NBA/LiCl) $C_{26}H_{38}O_8$ (478), 485 ($M+Li^+$)

Beispiel 12F

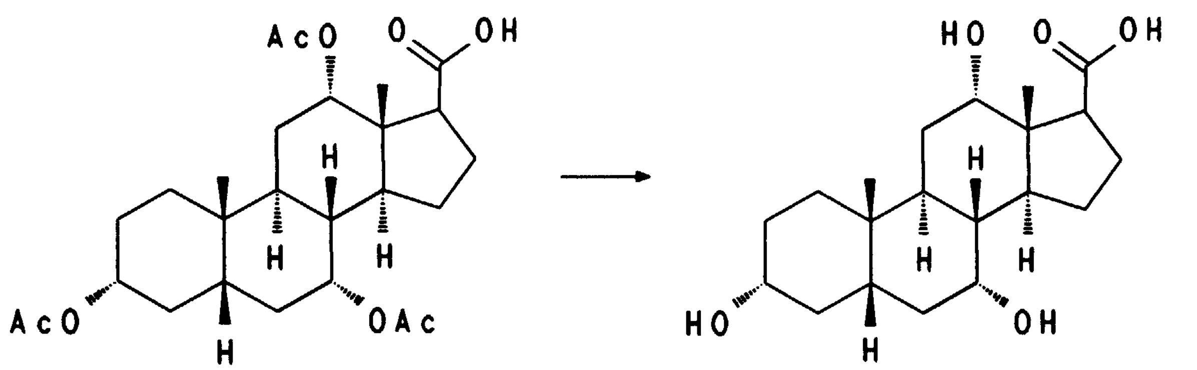

550 mg (1,15 mmol) Beispiel 12E werden in 20 ml Ethanol gelöst, mit 10 ml 2N NaOH versetzt und 24 Stunden bei Zimmertemperatur gerührt. Es wird Wasser zugegeben und die organischen Lösungsmittel abgezogen. Der pH-Wert wird mit 2N HCl auf 3 bis 4 eingestellt. Danach wird vollständig eingeengt und über Kieselgel chromatographiert ($CHCl_3$/MeOH = 4:1). Es werden 270 mg (67 %) Produkt erhalten.
MS (FAB, 3-NBA/LiCl) $C_{20}H_{32}O_5$ (352), 359 ($M+Li^+$)

Beispiel 13

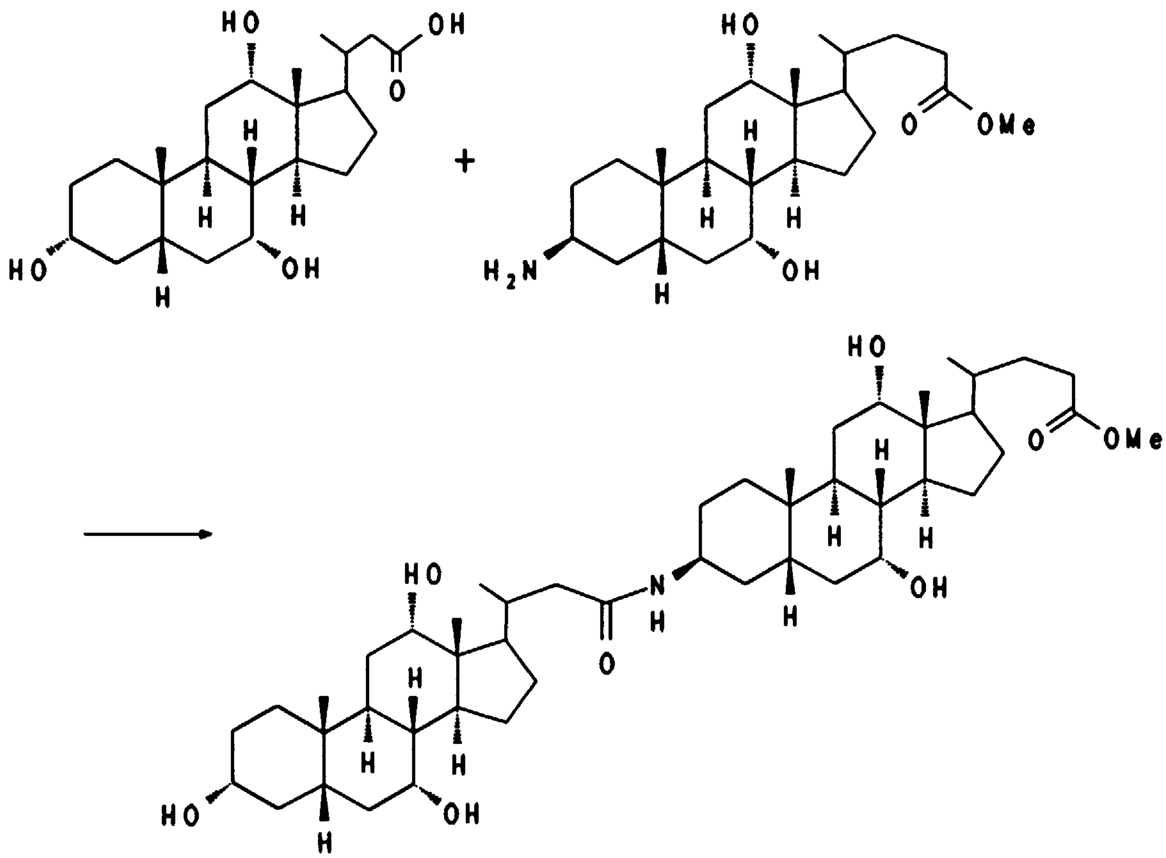

2,0 g (5,01 mmol) 3α,7α,12α-Trihydroxy-24-nor-23-cholansäure, 2,1 g (4,98 mmol) 3β-Amino-7α,12α-dihydroxy-24-cholansäuremethylester (vgl. EP-A-0 417 725), 1,36 g (10 mmol) Hydroxybenzotriazol und 1,04 g (5,4 mmol) Dicyclohexylcarbodiimid werden in 100 ml trockenem Tetrahydrofuran 24 Stunden bei Zimmertemperatur gerührt. Das Reaktionsgemisch wird eingeengt und der Rückstand über Kieselgel chromatographiert (Chloroform/Methanol = 85: 15). Es werden 3,0 g (75 %) Beispiel 13 erhalten.
MS (FAB, 3-NBA/LiCl) $C_{48}N_{79}NO_8$ (798), 805 ($M+Li^+$)

Analog zu Beispiel 13 werden die Beispiele 14 bis 31 der Tabellen 1 bis 3 erhalten (reaktionsfähige -X-G2-Derivate sind in EP-A-0 489 423 oder EP-A-0 417 725 beschrieben).

Tabelle 1:

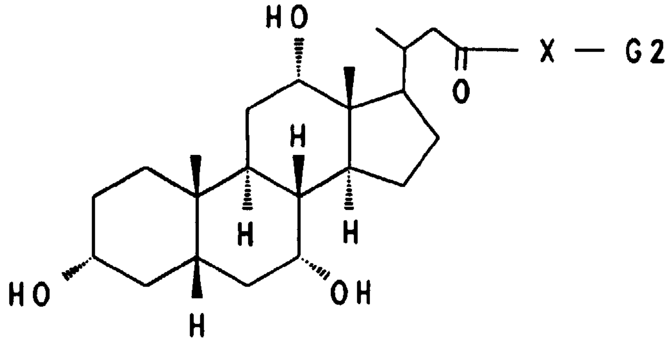

| Bsp. | - X - G2 | MS (FAB, 3-NBA/LiCl) |
|---|---|---|
| 14 | 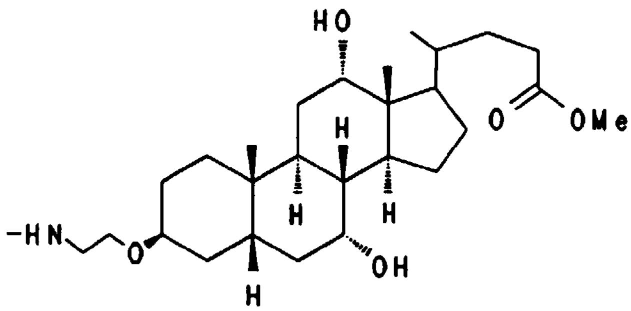 | $C_{50}H_{83}NO_9$ (842), 849 $(M+Li^+)$ |
| 15 | 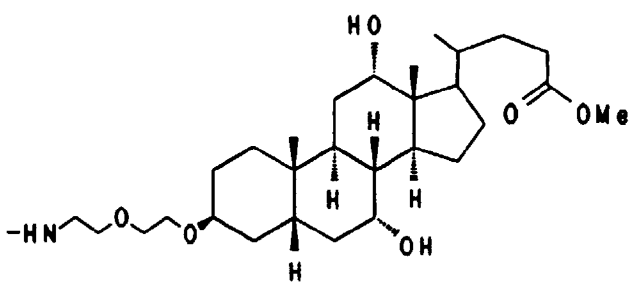 | $C_{52}H_{87}NO_{10}$ (886), 893 $(M+Li^+)$ |

| Bsp. | - X - G2 | MS (FAB, 3-NBA/LiCl) |
|---|---|---|
| 16 | 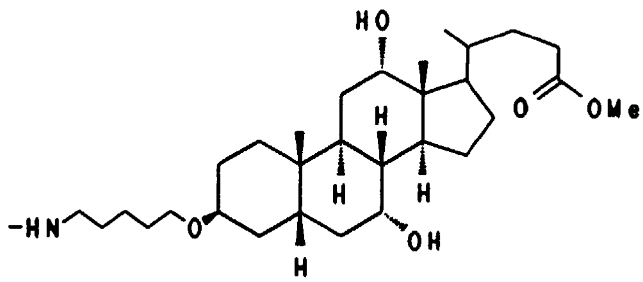 | $C_{54}H_{91}NO_9$ (898), 905 (M+Li$^+$) |
| 17 | 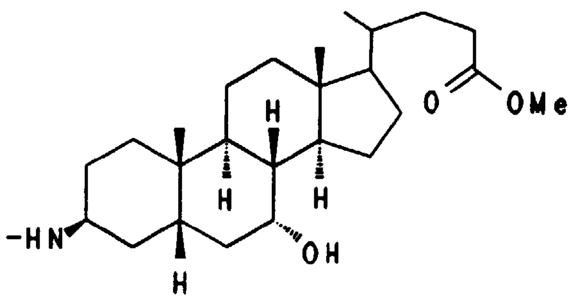 | $C_{48}H_{79}NO_7$ (782), 789 (M+Li$^+$) |
| 18 | 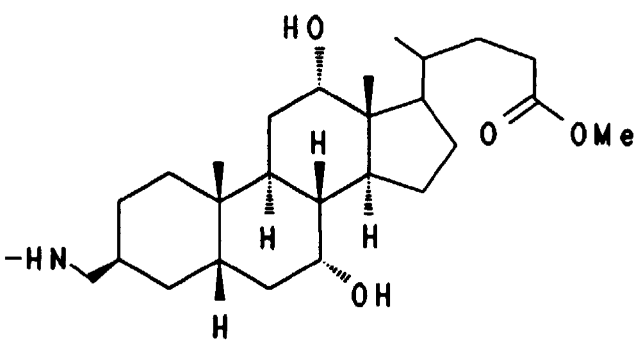 | $C_{49}H_{81}NO_8$ (812), 819 (M+Li$^+$) |

| Bsp. | - X - G2 | MS (FAB, 3-NBA/LiCl) |
|---|---|---|
| 19 | 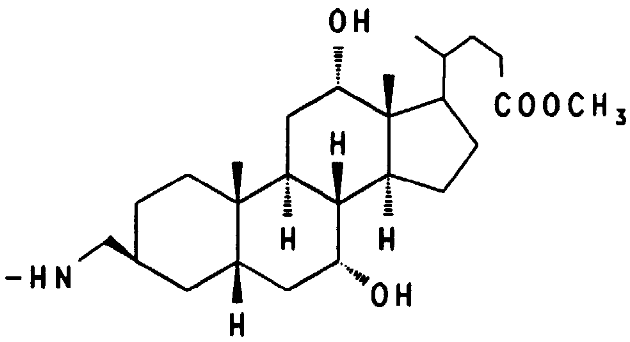 | $C_{55}H_{97}NO_9SI_2$ (972), 979 ($M+Li^+$) |

**Tabelle 2:**

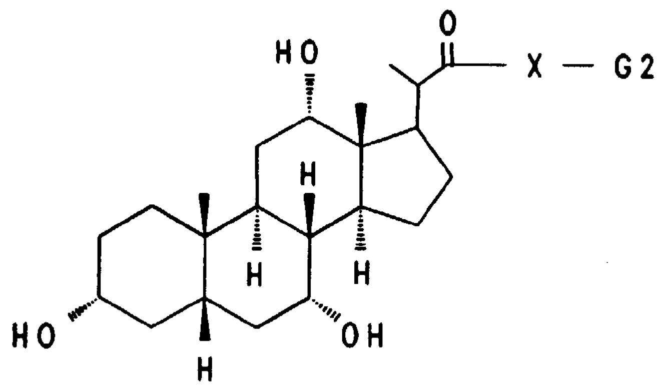

| Bsp. | - X - G2 | MS (FAB, 3-NBA/LiCl) |
|---|---|---|
| 20 | 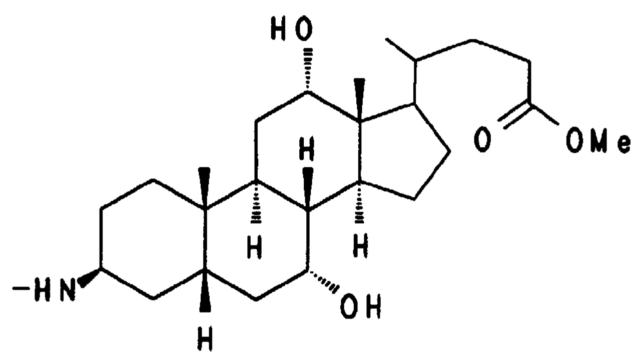 | $C_{47}H_{77}NO_8$ (784), 791 ($M+Li^+$) |
| 21 | 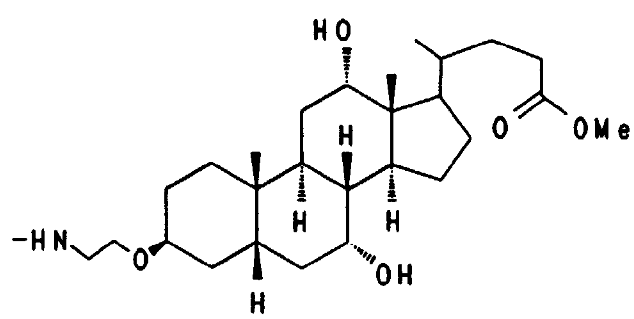 | $C_{49}H_{81}NO_9$ (828), 835 ($M+Li^+$) |

| Bsp. | - X - G2 | MS (FAB, 3-NBA/LiCl) |
|---|---|---|
| 22 | 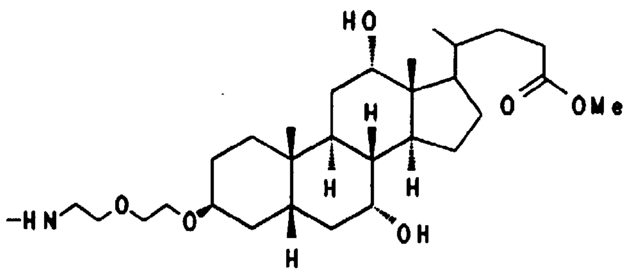 | $C_{51}H_{85}NO_{10}$ (872), 879 (M+Li$^+$) |
| 23 | 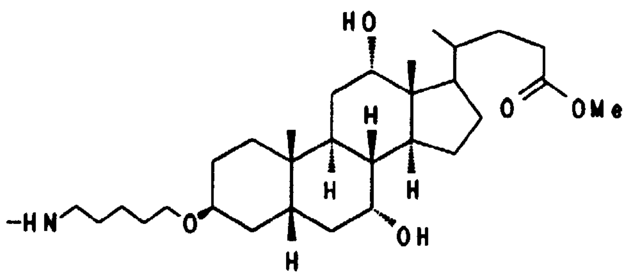 | $C_{53}H_{89}NO_9$ (884), 891 (M+Li$^+$) |
| 24 | 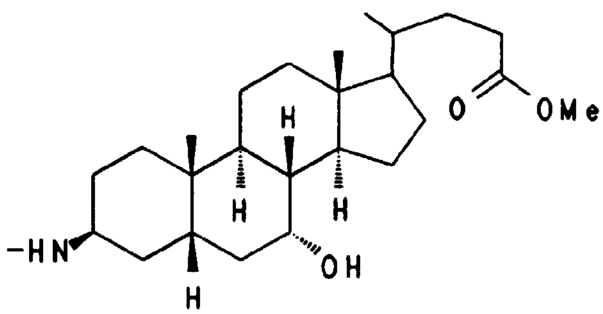 | $C_{47}H_{77}NO_7$ (768), 775 (M+Li$^+$) |

| Bsp. | - X - G2 | MS (FAB, 3-NBA/LiCl) |
|---|---|---|
| 25 | 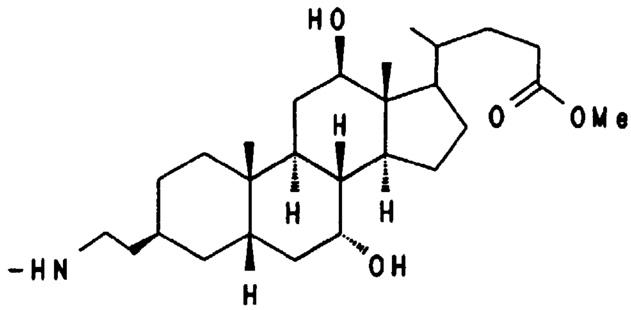 | $C_{49}H_{81}NO_8$ (812), 819 (M+Li$^+$) |
| 26 | 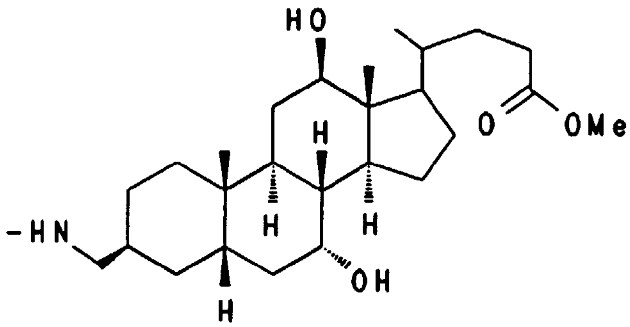 | $C_{48}H_{79}NO_9$ (814), 821 (M+Li$^+$) |

Tabelle 3

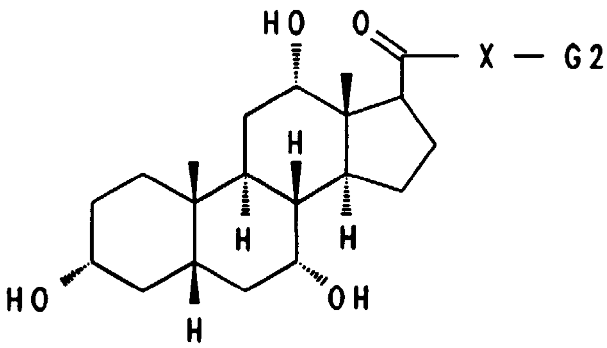

| Bsp. | - X - G2 | MS (FAB, 3-NBA/LiCl) |
|---|---|---|
| 27 | 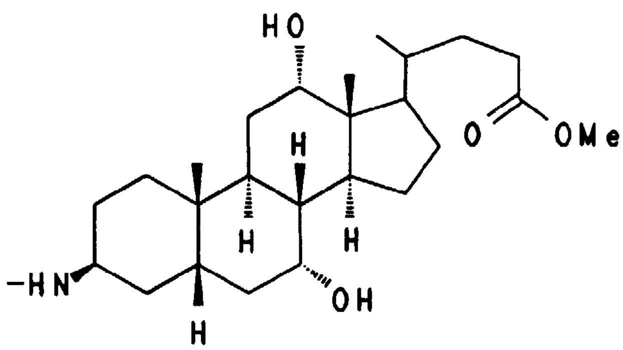 | $C_{45}H_{73}NO_8$ (756), 763 (M+Li$^+$) |
| 28 | 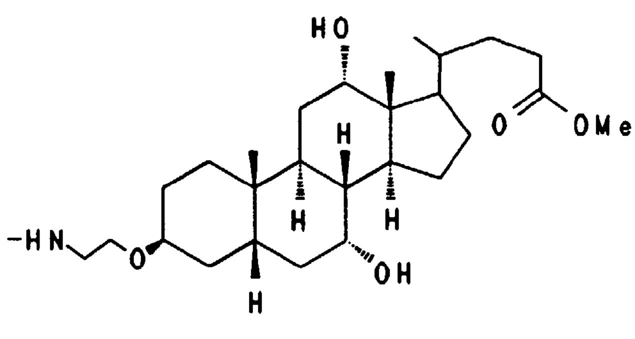 | $C_{47}H_{77}NO_9$ (800), 807 (M+Li$^+$) |

| Bsp. | - X - G2 | MS (FAB, 3-NBA/LiCl) |
|---|---|---|
| 29 | 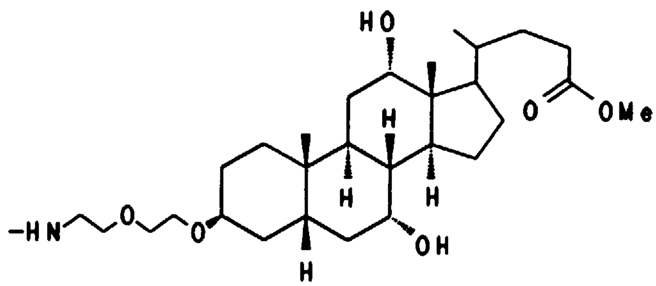 | $C_{49}H_{81}NO_{10}$ (844), 851 (M+Li$^+$) |
| 30 | 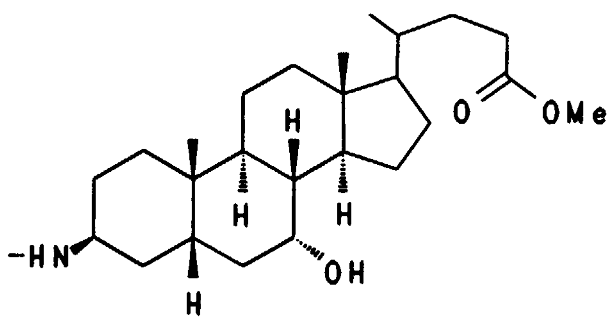 | $C_{45}H_{73}NO_7$ (740), 747 (M+Li$^+$) |
| 31 | 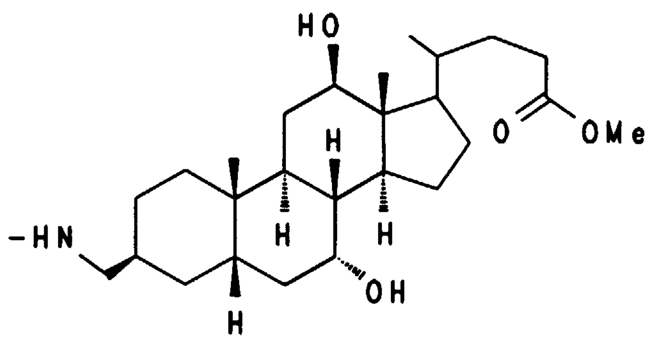 | $C_{46}H_{75}NO_7$ (754), 761 (M+Li$^+$) |

Beispiel 32

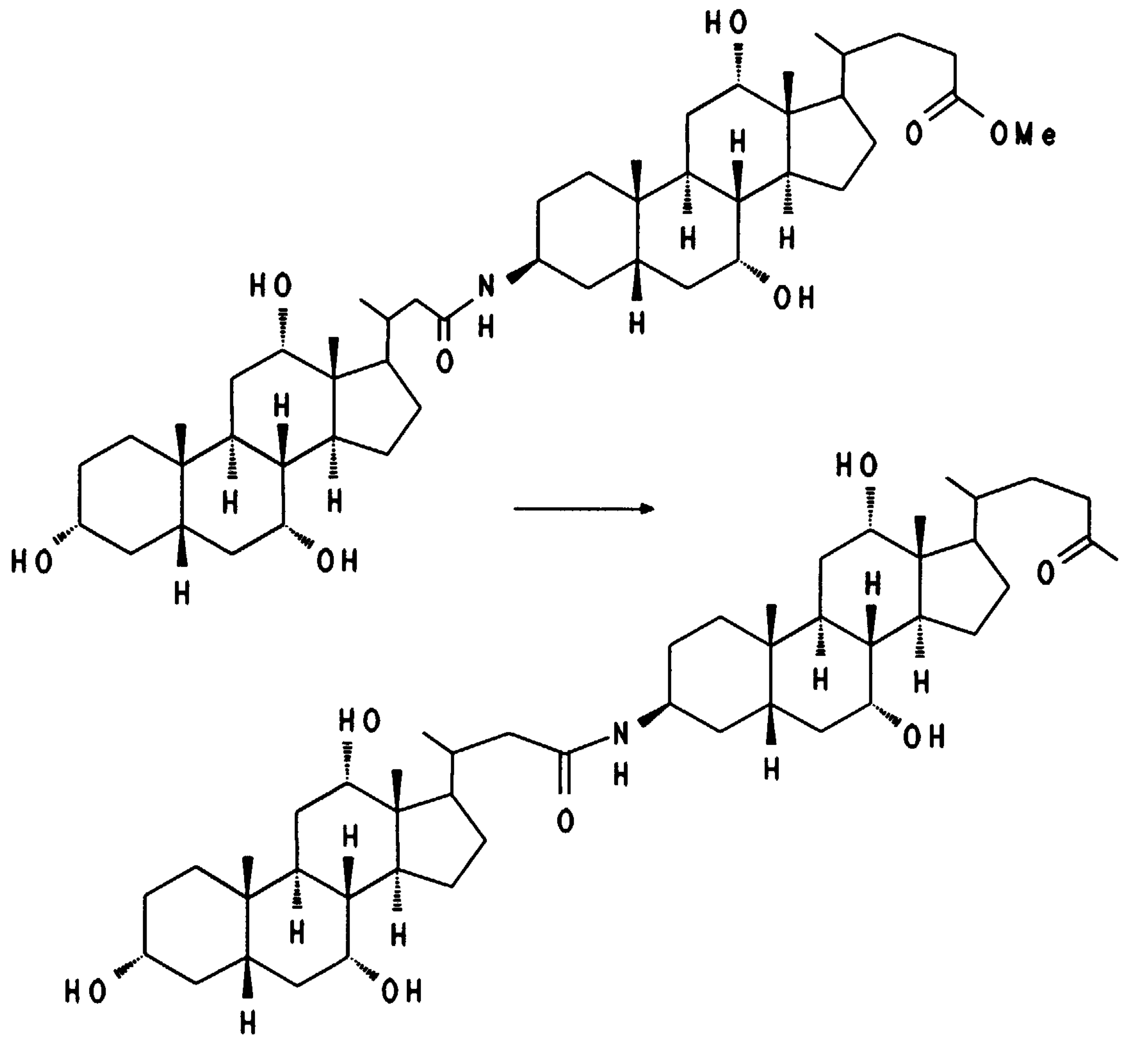

3,0 g (3,76 mmol) Beispiel 13 werden in 80 ml Ethanol gelöst, mit 30 mol IN wässriger NaOH versetzt und 16 Stunden bei Zimmertemperatur gerührt. Zur Aufarbeitung werden 30 ml Wasser zugegeben und der Alkohol vollständig abgezogen. Nach dem Ansäuern mit 1N HCl wird der Niederschlag abgesaugt, mit Wasser gewaschen und im Vakuum getrocknet. Es werden 2,5 g (85 %) Beispiel 32 erhalten.

MS (FAB, 3-NBA/LiCl) $C_{47}H_{77}NO_8$ (784), 791 ($M+Li^+$)

Analog zu Beispiel 32 werden aus den Methylestern (Tabellen 1 - 3) die Beispiele 33 bis 50 der Tabelle 4 bis 6 erhalten.

**Tabelle 4:**

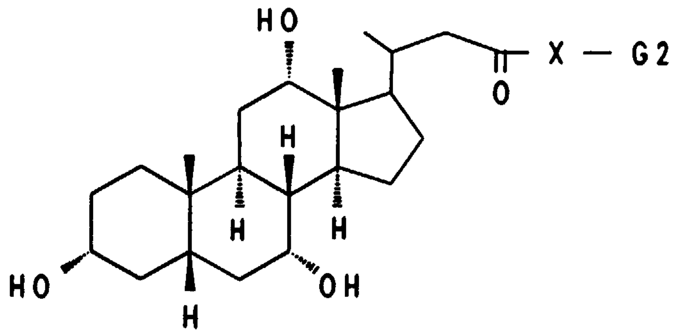

| Bsp. | - X - G2 | MS (FAB, 3-NBA/LiCl) |
|---|---|---|
| 33 | 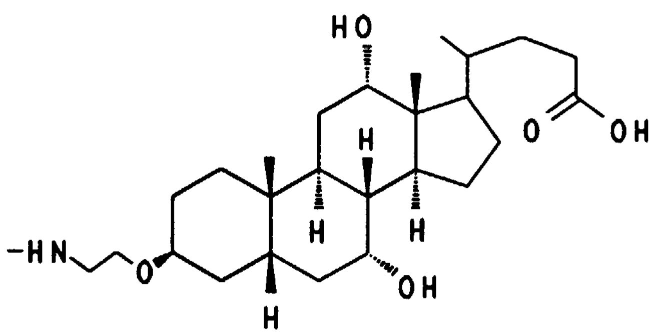 | $C_{49}H_{81}NO_9$ (828), 835 $(M+Li^+)$ |
| 34 | 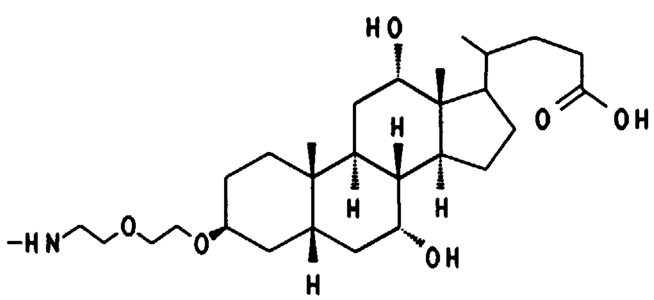 | $C_{51}H_{85}NO_{10}$ (872), 879 $(M+Li^+)$ |

| Bsp. | - X - G2 | MS (FAB, 3-NBA/LiCl) |
|---|---|---|
| 35 | 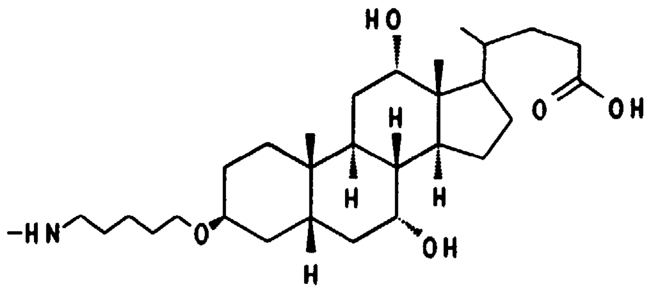 | $C_{53}H_{89}NO_9$ (884), 891 $(M+Li^+)$ |
| 36 | 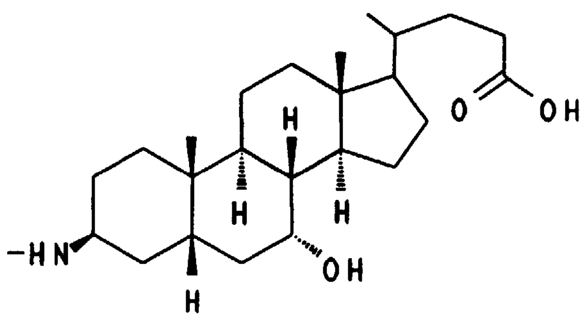 | $C_{47}H_{77}NO_7$ (768), 775 $(M+Li^+)$ |
| 37 | 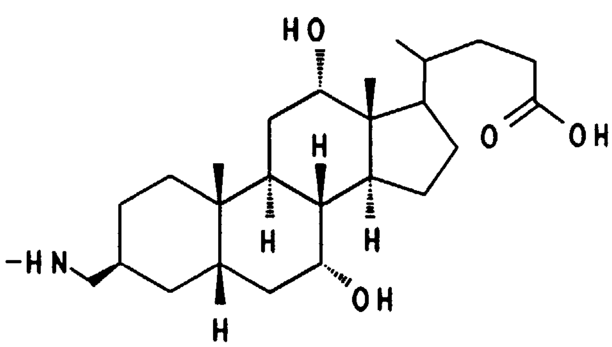 | $C_{48}H_{79}NO_8$ (798), 805 $(M+Li^+)$ |

| Bsp. | - X - G2 | MS (FAB, 3-NBA/LiCl) |
|---|---|---|
| 38 | 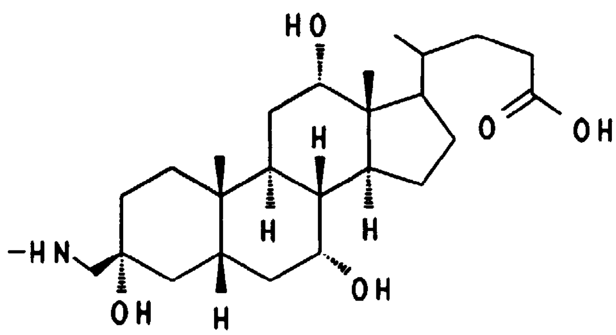 | $C_{48}H_{79}NO_9$ (814), 821 (M+Li$^+$) |

Tabelle 5

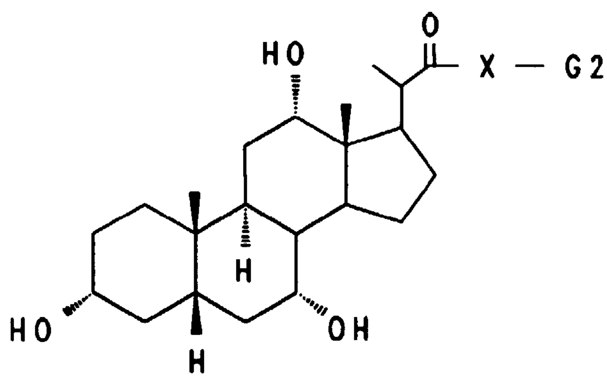

| Bsp. | - X - G2 | MS (FAB, 3-NBA/LiCl) |
|---|---|---|
| 39 | 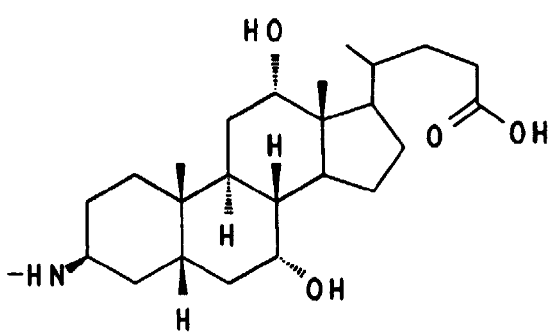 | $C_{46}H_{75}NO_8$ (770), 777 (M+Li$^+$) |

| Bsp. | - X - G2 | MS (FAB, 3-NBA/LiCl) |
| --- | --- | --- |
| 40 | 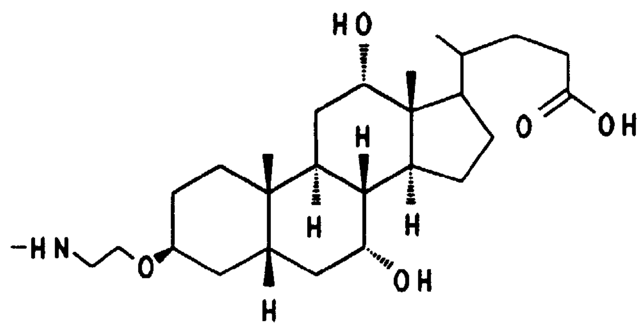 | $C_{48}H_{79}NO_9$ (814), 821 (M+Li$^+$) |
| 41 | 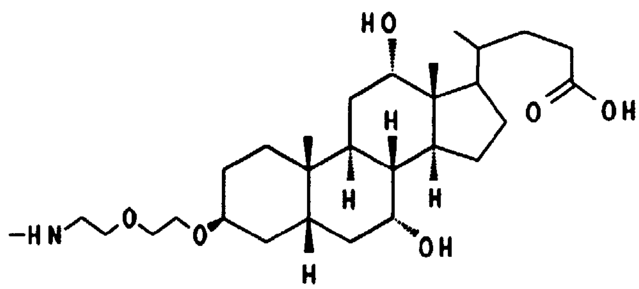 | $C_{50}H_{83}NO_{10}$ (858), 865 (M+Li$^+$) |
| 42 | 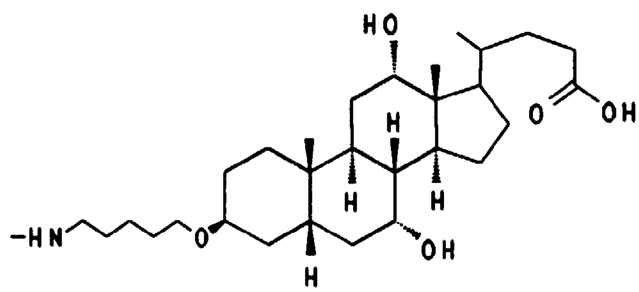 | $C_{52}H_{87}NO_9$ (870), 877 (M+Li$^+$) |

| Bsp. | - X - G2 | MS (FAB, 3-NBA/LiCl) |
|---|---|---|
| 43 | 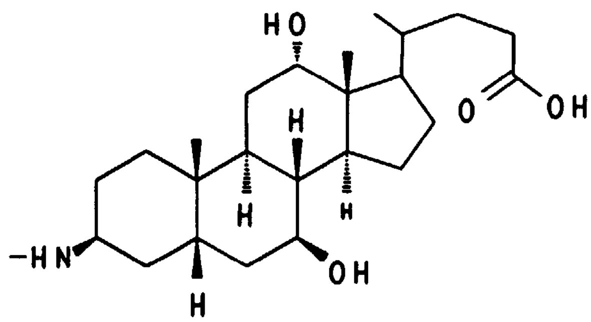 | $C_{46}H_{75}NO_7$ (754), 755 ($M+Li^+$) |
| 44 | 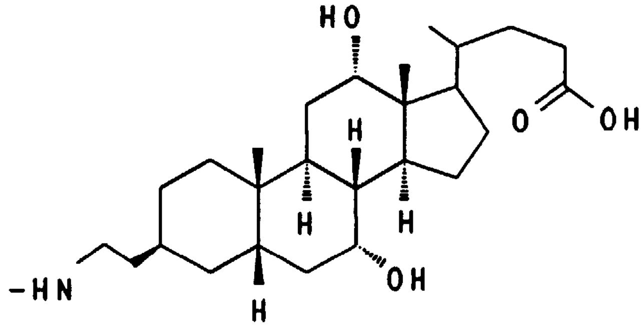 | $C_{48}H_{79}NO_8$ (798), 805 ($M+Li^+$) |
| 45 | 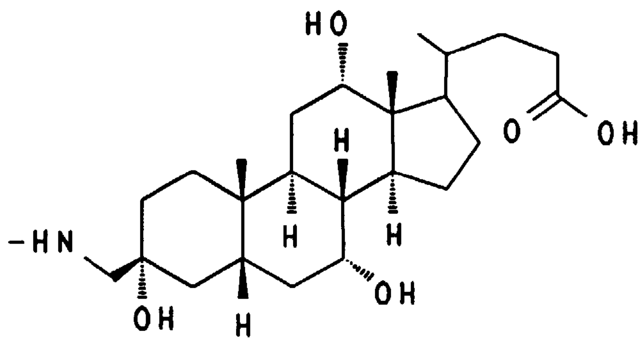 | $C_{47}H_{77}NO_9$ (800), 807 ($M+Li^+$) |

Tabelle 6:

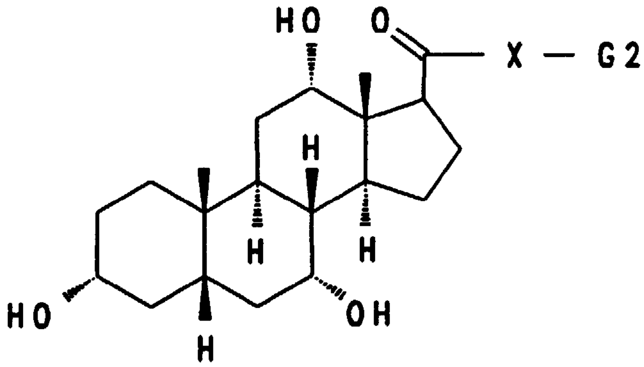

| Bsp. | - X - G2 | MS (FAB, 3-NBA/LiCl) |
|---|---|---|
| 46 | 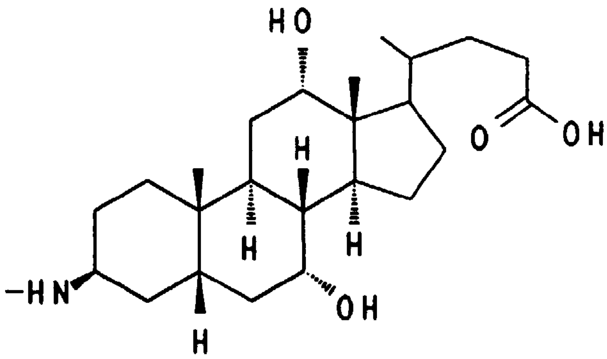 | $C_{44}H_{71}NO_8$ (742), 749 $(M+Li^+)$ |
| 47 | 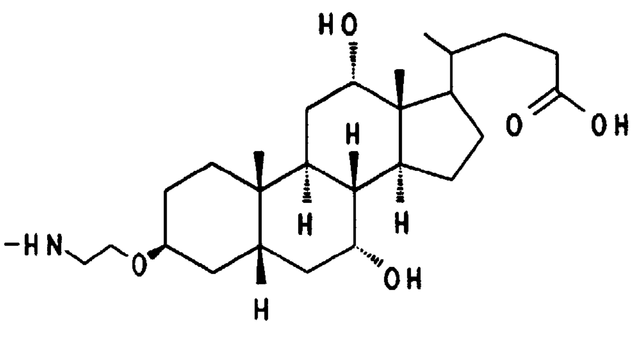 | $C_{46}H_{75}NO_9$ (786), 793 $(M+Li^+)$ |

| Bsp. | - X - G2 | MS (FAB, 3-NBA/LiCl) |
|---|---|---|
| 48 | 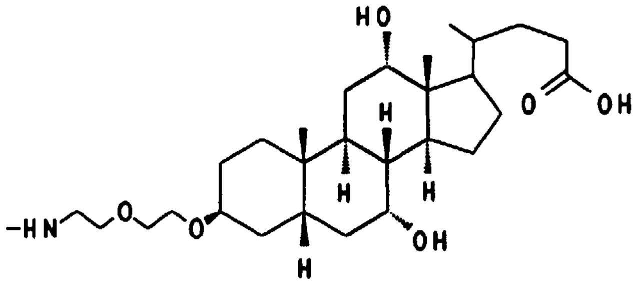 | $C_{48}H_{79}NO_{10}$ (830), 837 (M+Li$^+$) |
| 49 | 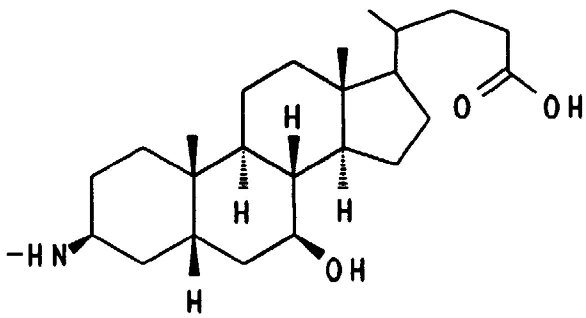 | $C_{44}H_{71}NO_7$ (726), 733 (M+Li$^+$) |
| 50 | 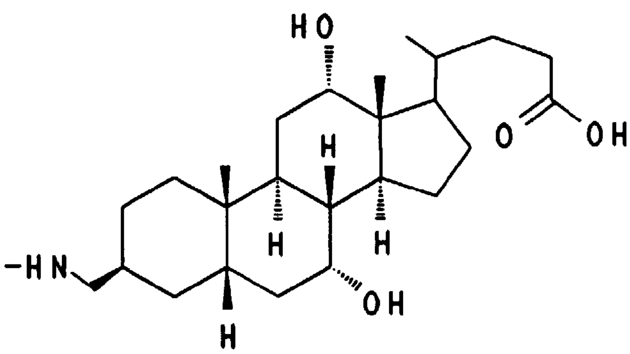 | $C_{45}H_{73}NO_7$ (740), 747 (M+Li$^+$) |

Analog zu Beispiel 5 werden aus vorstehend beschriebenen Säuren die Beispiele 51 bis 54 der Tabelle 7 erhalten.

Tabelle 7:

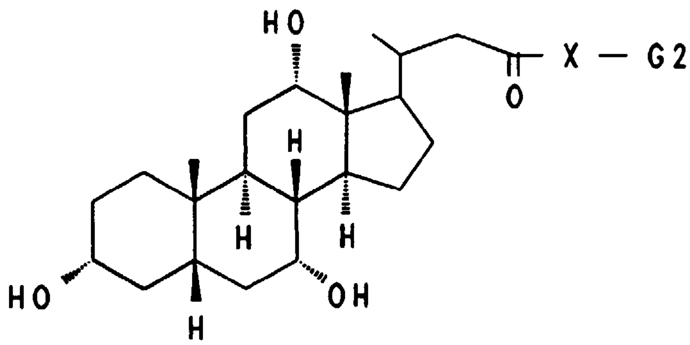

| Bsp. | - X - G2 | MS (FAB, 3-NBA/LiCl) |
|---|---|---|
| 51 | 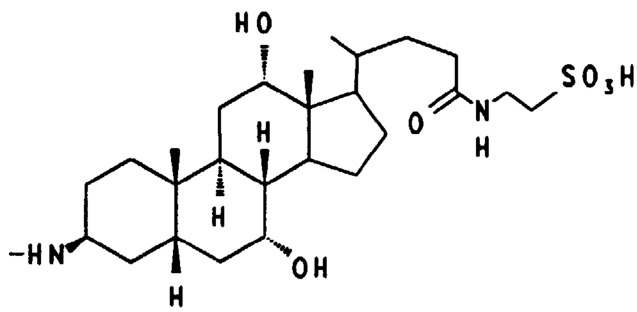 | $C_{49}H_{82}N_2O_{10}S$ (891), 892 $(M+H^+)$ |
| 52 | 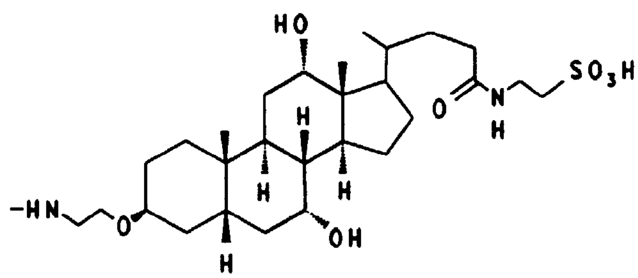 | $C_{51}H_{86}N_2O_{11}S$ (935), 942 $(M+H^+)$ |

| Bsp. | - X - G2 | MS (FAB, 3-NBA/LiCl) |
|---|---|---|
| 53 | 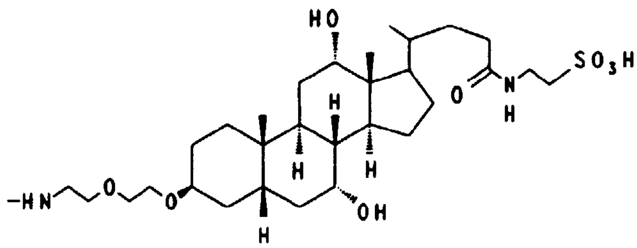 | $C_{53}H_{90}N_2O_{12}S$ (979), 1024 $(M+H^+)$ |
| 54 | 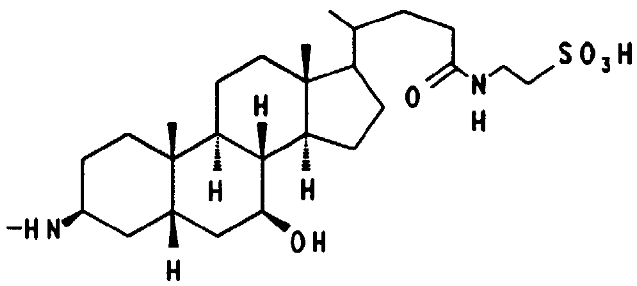 | $C_{49}H_{82}N_2O_9S$ (875), 920 $(M+H^+)$ |

Analog zu Beispiel 4 werden die Beispiele 55 bis 57 der Tabelle 8 erhalten.

Tabelle 8:

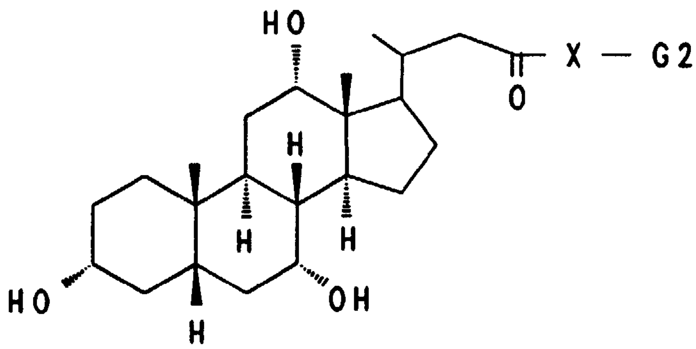

| Bsp. | - X - G2 | MS (FAB, 3-NBA/LiCl) |
|---|---|---|
| 55 | 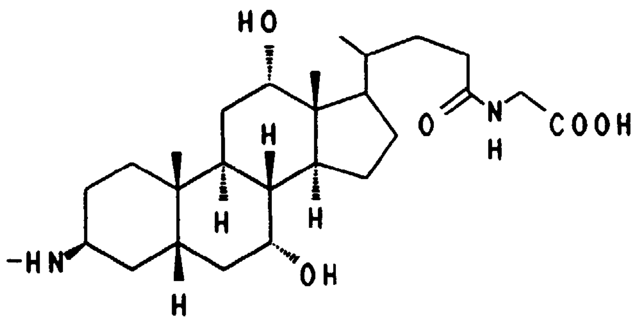 | $C_{49}H_{80}N_2O_9$ (841), 842 ($M+H^+$) |
| 56 | 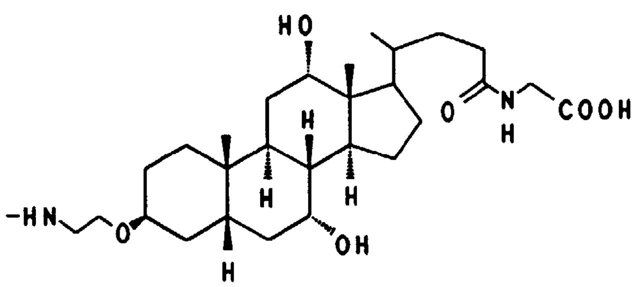 | $C_{51}H_{84}N_2O_{10}$ (885), 892 ($M+Li^+$) |

| Bsp. | - X - G2 | MS (FAB, 3-NBA/LiCl) |
|---|---|---|
| 57 | 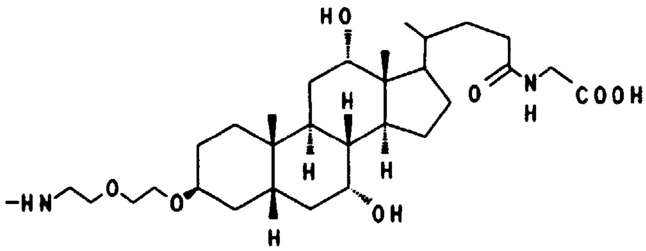 | $C_{53}H_{88}N_2O_{11}$ (929), 936 $(M+Li^+)$ |

Analog zu Beispiel 13 werden die Beispiele 58 bis 63 der Tabelle 9 erhalten.

**Tabelle 9:**

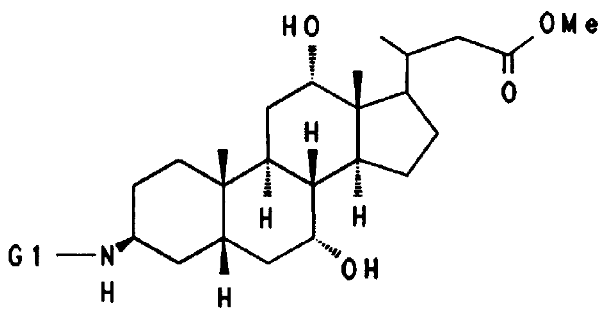

**(in den folgenden Formeln ist die freie Valenz von G1 nicht gezeichnet)**

| Bsp. | G1 - | MS (FAB, 3-NBA/LiCl) |
|---|---|---|
| 58 | 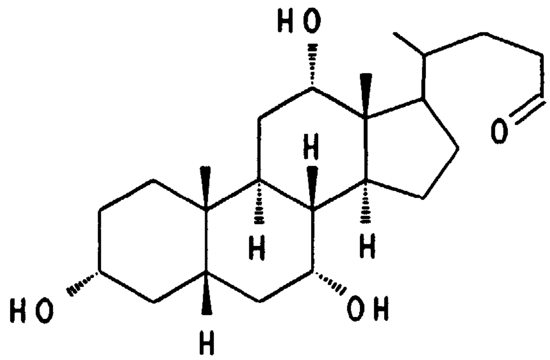 | $C_{48}H_{79}NO_8$ (798), 805 (M+Li$^+$) |
| 59 | 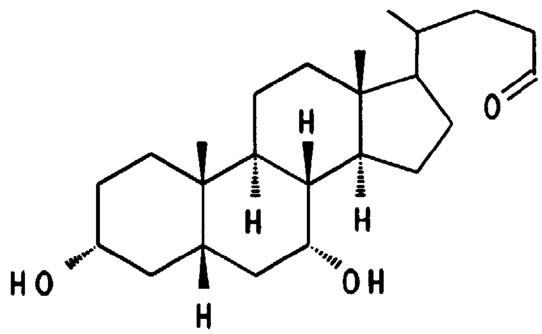 | $C_{48}H_{79}NO_7$ (782), 789 (M+Li$^+$) |
| 60 | 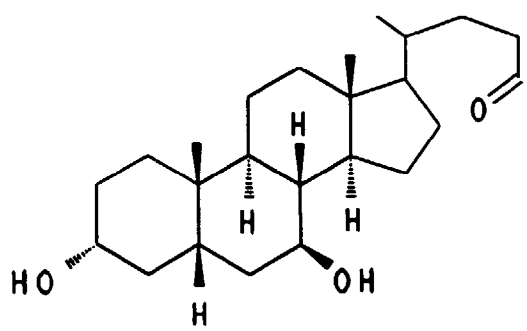 | $C_{48}H_{79}NO_8$ (782), 789 (M+Li$^+$) |

| Bsp. | G1 - | MS (FAB, 3-NBA/LiCl) |
|---|---|---|
| 61 | 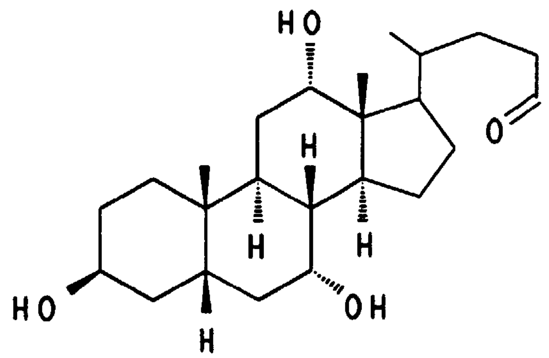 | $C_{48}H_{79}NO_8$ (798), 805 (M+Li$^+$) |
| 62 | 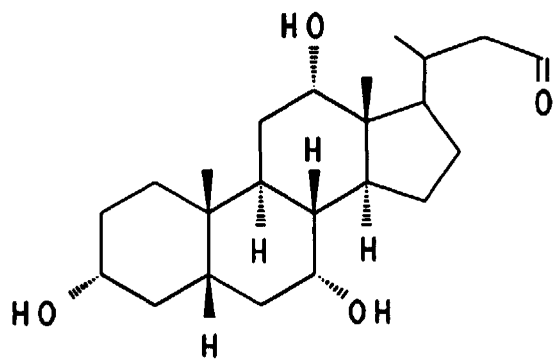 | $C_{47}H_{77}NO_8$ (784), 791 (M+Li$^+$) |
| 63 | 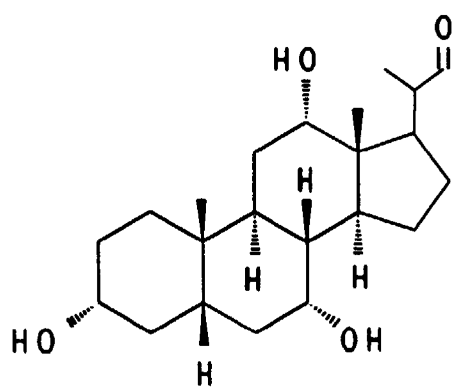 | $C_{46}H_{75}NO_8$ (770), 777 (M+Li$^+$) |

Analog zu Beispiel 32 werden die Beispiele 64 bis 69 der Tabelle 10 erhalten.

Tabelle 10:

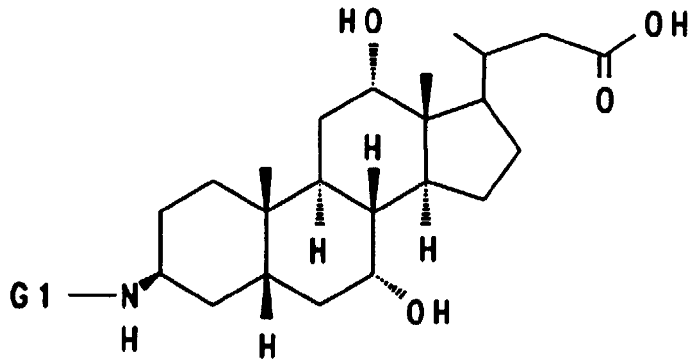

(in den folgenden Formeln ist die freie Valenz von G1 nicht gezeichnet)

| Bsp. | G1 - | MS (FAB, 3-NBA/LiCl) |
|---|---|---|
| 64 | 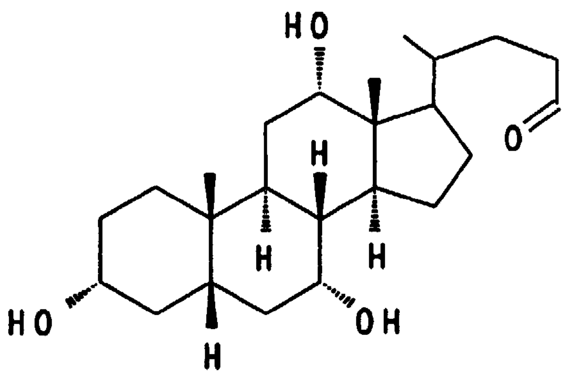 | $C_{47}H_{77}NO_8$ (784), 791 (M+Li$^+$) |
| 65 | 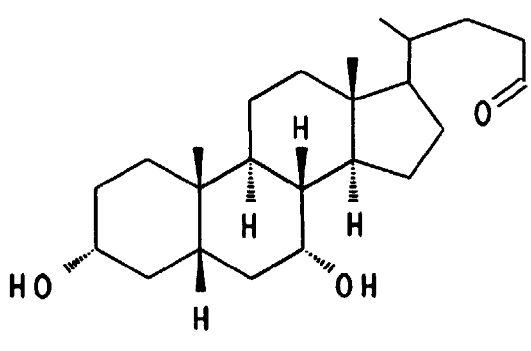 | $C_{47}H_{77}NO_7$ (768), 775 (M+Li$^+$) |

| Bsp. | G1 - | MS (FAB, 3-NBA/LiCl) |
|---|---|---|
| 66 | 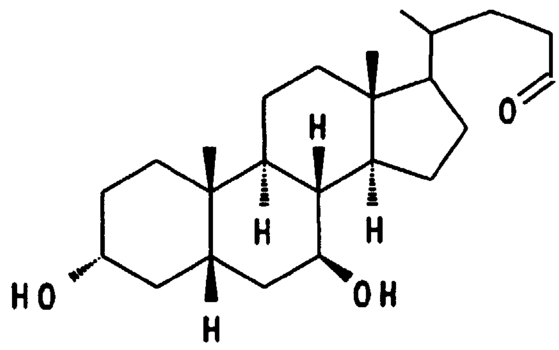 | $C_{47}H_{77}NO_7$ (768), 775 $(M+Li^+)$ |
| 67 | 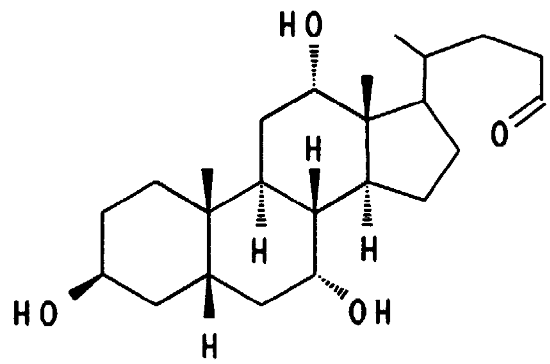 | $C_{47}H_{77}NO_8$ (784), 791 $(M+Li^+)$ |
| 68 | 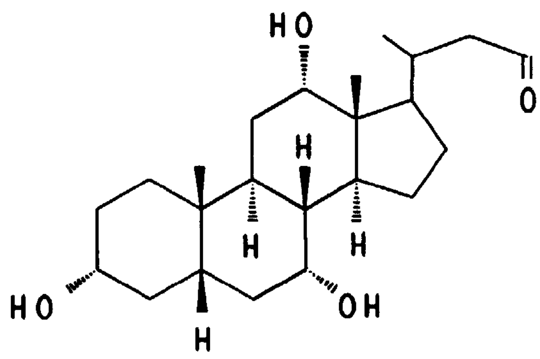 | $C_{46}H_{75}NO_8$ (770), 771 $(M+Li^+)$ |

| Bsp. | G1 - | MS (FAB, 3-NBA/LiCl) |
|---|---|---|
| 69 | 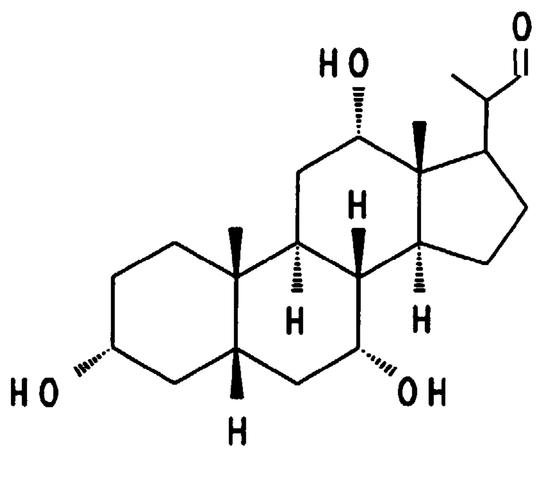 | $C_{45}H_{73}NO_8$ (756), 763 (M+Li$^+$) |

Von Beispiel 32 und allen Beispielen der Tabellen 4 bis 8 und 10 können die Natriumsalze hergestellt werden. Die Verbindung wird in Methanol gelöst, mit einer äquimolaren Menge 1N wässriger NaOH versetzt und anschließend im Vakuum eingedampft.

Tabelle 11 zeigt Meßwerte der Hemmung der [$^3$H]-Taurocholataufnahme in Bürstensaummembranvesikel des Ileums von Kaninchen. Angegeben sind die Quotienten aus den $IC_{50}$- bzw. $IC_{50Na}$-Werten der Referenzsubstanz als Taurochenodesoxycholat (TCDC) und der jeweiligen Testsubstanz.

Tabelle 11:

| Verbindungen aus Beispiel | $\frac{IC_{50}\text{-TCDC}[\mu mol]}{IC_{50}\text{-Substanz}[\mu mol]}$ | $\frac{IC_{50Na}\text{-TCDC}[\mu mol]}{IC_{50Na}\text{-Substanz}[\mu mol]}$ |
|---|---|---|
| 32 | 0,76 | 0,65 |
| 35 | 1,00 | 0,91 |
| 36 | 0,78 | 1,14 |
| 41 | 0,55 | 0,58 |
| 43 | 0,69 | 0,78 |
| 57 | 0,34 | 0,34 |
| 65 | 0,85 | 0,84 |
| 69 | 0,78 | 0,86 |

**Patentansprüche**

1. Gallensäurederivate der Formel I

G1-X-G2    I

in der G1 über die Seitenkette am Ring D mit dem Verbindungsglied X an Ring A von G2 verknüpft ist, und G1 einen Rest der Formel II

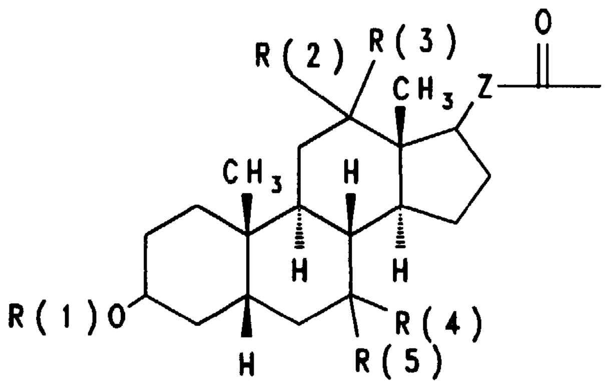

II

wobei

Z einen der folgenden Reste

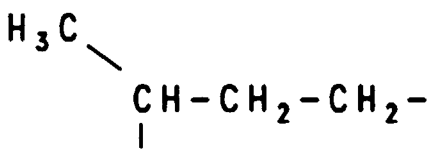 , 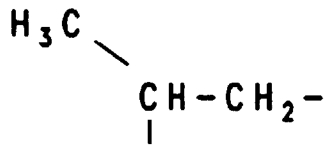 , 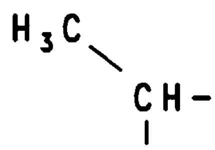 ,

oder eine einfache Bindung darstellt,

R(1) H, einen Alkylrest mit 1 bis 10 C-Atomen, einen Alkenylrest mit 2 bis 10 C-Atomen bedeutet,

R(2) bis R(5), wobei R(2) and R(3) bzw. R(4) and R(5) jeweils gemeinsam der Sauerstoff einer Carbonylgruppe oder jeweils unabhängig voneinander H oder OH bedeuten,

X eine Einfachbindung oder ein Brückenglied der Formel III ist,

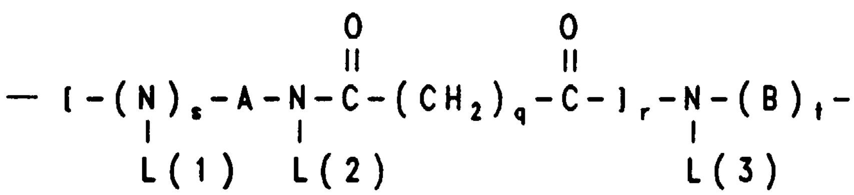

(III)

wobei

A eine Alkylenkette, die verzweigt oder unverzweigt ist und die Kette ggf. durch -O- oder -S- unterbrochen sein kann und 2 bis 6 Kettenglieder p umfaßt,

B eine Alkylenkette, die verzweigt oder unverzweigt ist und die Kette ggf. durch -O- oder -S- unterbrochen sein kann und die Kette 2 bis 6 Kettenglieder n umfaßt,

L(1), L(2), L(3) gleich oder verschieden sind und H, $C_1$-$C_4$-Alkyl oder Benzyl bedeuten, und

q 0 bis 5
r 0 oder 1
s 0 oder 1
t 0 oder 1

bedeuten, und

G2 einen Rest der Formel IV bedeutet

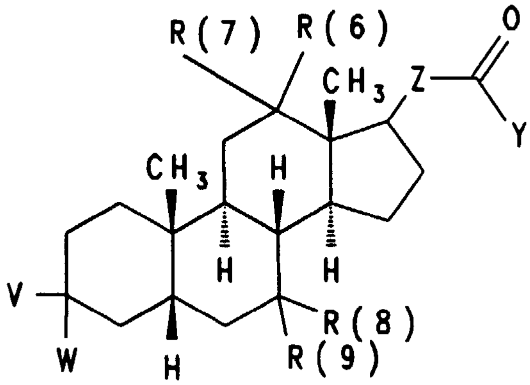

IV

wobei

Z einen der folgenden Reste

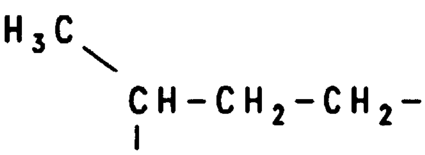 , 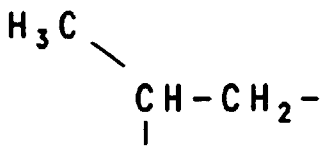 , 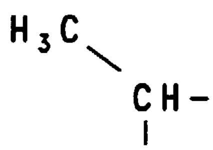 ,

oder eine einfach Bindung bedeutet,

V

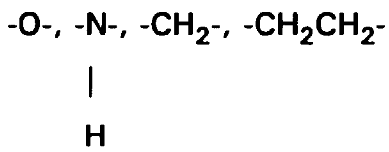

W H oder, falls V = $-CH_2-$, $-CH_2CH_2-$ ist, kann W H oder OH sein,

Y -OL, -NHL,

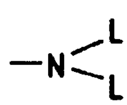

eine über die Aminogruppe gebundene Aminosäure oder Aminosulfonsäure, ausgewählt aus der Gruppe bestehend aus $-NH-CH_2-COOH$, $-NH-CH_2-CH_2-SO_3H$,

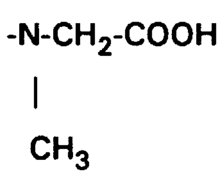 und 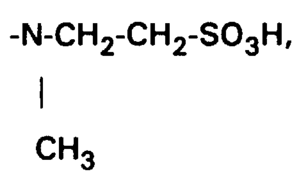,

wobei L

H, einen Alkylrest mit bis zu 10 C-Atomen, einen Alkenylrest mit 2 bis 10 C-Atomen, einen Cycloalkylrest mit 3 bis 8 C-Atomen, einen Phenylrest, der unsubstituiert ist oder 1- bis 3-fach substituiert ist mit F, Cl, Br, ($C_1$-$C_4$)-Alkyl oder ($C_1$-$C_4$)-Alkoxy, einen Benzylrest, der unsubstituiert oder 1 bis 3fach substituiert ist mit F, Cl, Br, ($C_1$-$C_4$)-Alkyl oder ($C_1$-$C_4$)-Alkoxy bedeutet, und
R(6) - R(9), wobei R(6) und R(7) bzw. R(8) und R(9) jeweils gemeinsam der Sauerstoff einer Carbonylgruppe oder einzeln und jeweils unabhängig voneinander H, -OH sind, und mindestens einer der beiden Gallensäurereste G1 oder G2 in der Seitenkette um ein oder mehrere C-Atome verkürzt ist, d.h. Z eine Bindung,

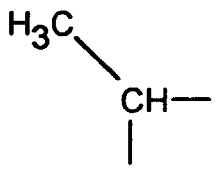

oder

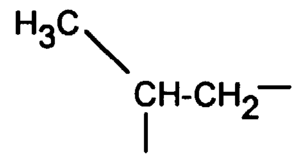

bedeutet.

2. Arzneimittel enthaltend ein Gallensäurederivat gemäß Anspruch 1.

3. Arzneimittel nach Anspruch 2 zur Behandlung von Hyperlipidämie.

4. Verwendung eines Gallensäurederivates gemäß Anspruch 1 zur Herstellung eines Medikaments zur Behandlung von Hyperlipidämie.

**Claims**

1. A bile acid derivative of the formula I

G1 - X - G2      I

in which G1 is linked via the side chain on the ring D to the connecting member X on ring A of G2, and G1 is a radical of the formula II

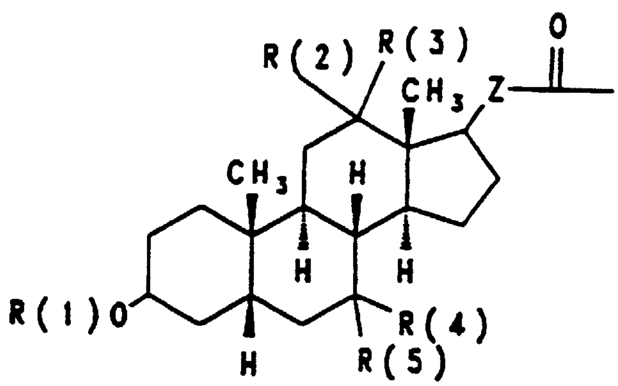

II

where

Z     is one of the following radicals

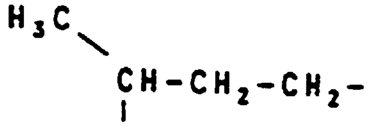,
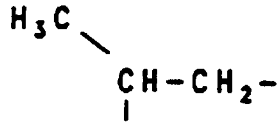,
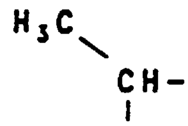,

or a single bond,

R(1) is H, an alkyl radical having 1 to 10 carbon atoms, an alkenyl radical having 2 to 10 carbon atoms,

R(2) and R(3) or R(4) and R(5) in each case together are the oxygen of a carbonyl group or in each case independently of one another are H or OH,

X is a single bond or a bridge member of the formula III,

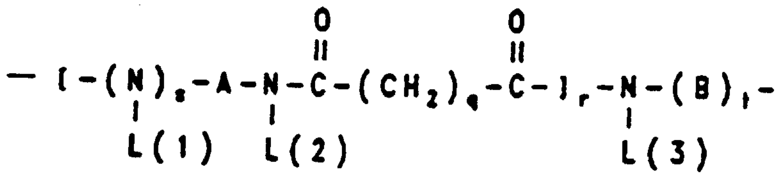
(III)

where

A is an alkylene chain which is branched or unbranched and the chain can optionally be interrupted by -O- or -S- and comprises 2 to 6 chain members p,

B is an alkylene chain which is branched or unbranched and the chain can optionally be interrupted by -O- or -S- and the chain comprises 2 to 6 chain members n,

L(1), L(2), L(3) are identical or different and are H, $C_1$-$C_4$-alkyl or benzyl, and

q is 0 to 5
r is 0 or 1
s is 0 or 1
t is 0 or 1, and

G2 is a radical of the formula IV

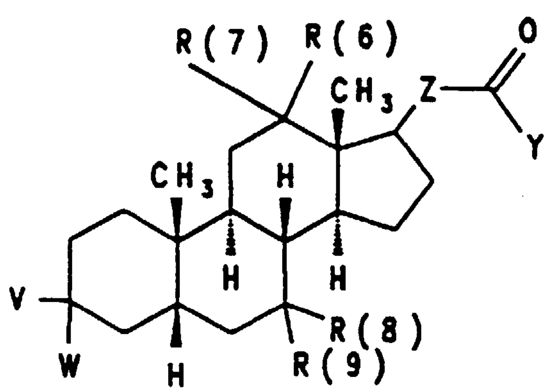
IV

where

Z is one of the following radicals

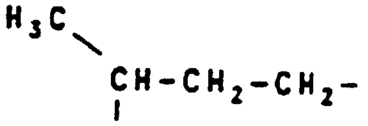, 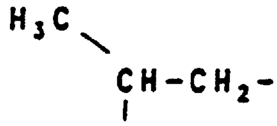, 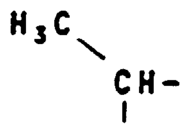,

or a single bond,

V is -O-,

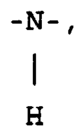

$-CH_2-$, $-CH_2CH_2-$

W is H or, if V = $-CH_2-$, $-CH_2CH_2-$, W can be H or OH,

Y is -OL, -NHL,

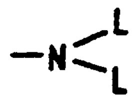

an amino acid or an aminosulfonic acid bonded via the amino group, selected from the group consisting of $-NH-CH_2-COOH$, $-NH-CH_2-CH_2-SO_3H$,

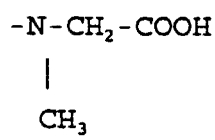

and

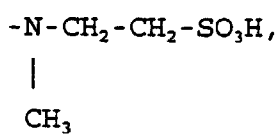

where L

is H, an alkyl radical having up to 10 carbon atoms, an alkenyl radical having 2 to 10 carbon atoms, a cycloalkyl radical having 3 to 8 carbon atoms, a phenyl radical which is unsubstituted or substituted 1 to 3 times by F, Cl, Br, ($C_1$-$C_4$)-alkyl or ($C_1$-$C_4$)-alkoxy, a benzyl radical which is unsubstituted or substituted 1 to 3 times by F, Cl, Br, ($C_1$-$C_4$)-alkyl or ($C_1$-$C_4$)-alkoxy, and

R(6) and R(7) or R(8) and R(9) in each case together are the oxygen of a carbonyl group or individually and in each case independently of one another are H, -OH, and at least one of the two bile acid radicals G1 or G2 in the side chain is shortened by one or more carbon atoms, i.e. Z is a bond,

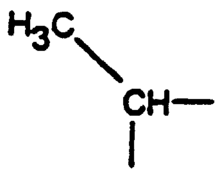

or

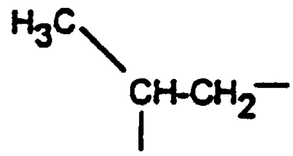 .

2. A pharmaceutical comprising a bile acid derivative as claimed in claim 1.

3. A pharmaceutical as claimed in claim 2 for the treatment of hyperlipidemia.

4. The use of a bile acid derivative as claimed in claim 1 for the production of a medicament for the treatment of hyperlipidemia.

## Revendications

1. Dérivés d'acides biliaires de formule I

G1 - X - G2 I

dans laquelle G1 est relié, par l'intermédiaire de la chaîne latérale sur le cycle D, par le chaînon de liaison X au cycle A de G2, et G1 représente un radical de formule II

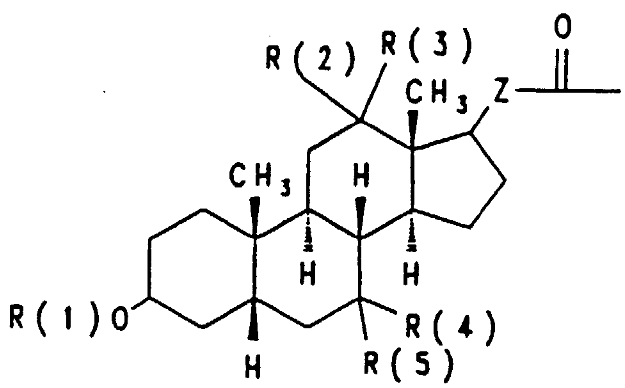

II

dans laquelle

Z représente l'un des radicaux suivants

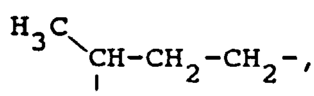 , 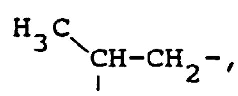 , 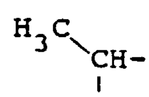

ou une liaison simple,

R(1) représente H ou un radical alkyle ayant de 1 à 10 atomes de carbone, un radical alcényle ayant de 2 à 10 atomes de carbone,

R(2) à R(5) représentent chacun, indépendamment les uns des autres, H ou OH, ou bien R(2) et R(3) ou, respectivement, R(4) et R(5) représentent ensemble l'atome d'oxygène d'un groupe carbonyle,

X est une liaison simple ou un chaînon pontant de formule III

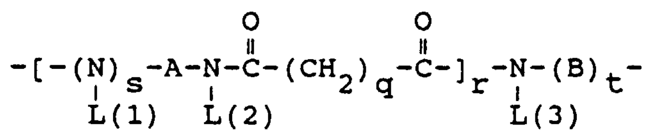 (III)

A représentant une chaîne alkylène ramifiée ou non ramifiée et la chaîne pouvant éventuellement être interrompue par -O- ou -S- et comprenant 2 à 6 chaînons p formant la chaîne,

B représentant une chaîne alkylène ramifiée ou non ramifiée et la chaîne pouvant éventuellement être interrompue par -O- ou -S- et la chaîne comprenant 2 à 6 chaînons n formant la chaîne,

L(1), L(2), L(3) étant identiques ou différents et représentant H ou un groupe alkyle en $C_1$-$C_4$ ou benzyle, et

q allant de 0 à 5,

r étant 0 ou 1,

s étant 0 ou 1,

t étant 0 ou 1, et

G2 représente un radical de formule IV

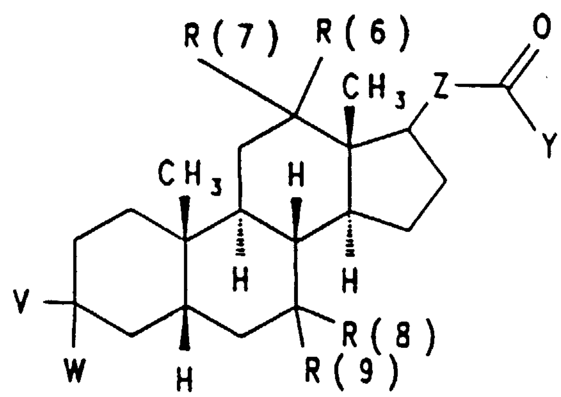 IV

dans laquelle

Z représente l'un des radicaux suivants

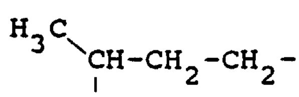 , 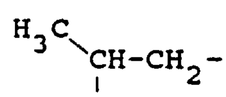 , 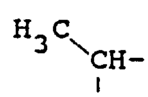

ou une liaison simple,

V représente -O-,

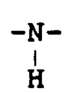 ,

$-CH_2-$, $-CH_2CH_2-$ ,

W représente H ou, lorsque V est $-CH_2-$ ou $-CH_2CH_2-$, W peut être H ou OH,

Y représente -OL, -NHL,

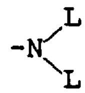

un aminoacide ou un acide aminosulfonique relié par le groupe amino, choisi parmi $-NH-CH_2-COOH$, $-NH-CH_2-CH_2-SO_3H$,

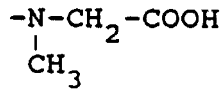 et 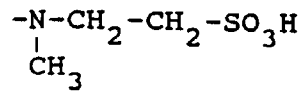

L représentant
H ou un radical alkyle ayant jusqu'à 10 atomes de carbone, un radical alcényle ayant de 2 à 10 atomes de carbone, un radical cycloalkyle ayant de 3 à 8 atomes de carbone, un radical phényle non substitué ou 1 à 3 fois substitué par un ou des atomes de F, Cl, Br ou groupes alkyle en $C_1$-$C_4$ ou alcoxy en $C_1$-$C_4$, un radical benzyle non substitué ou 1 à 3 fois substitué par un ou des atomes de F, Cl, Br ou groupes alkyle en $C_1$-$C_4$ ou alcoxy en $C_1$-$C_4$, et

R(6) à R(9) représentent chacun, indépendamment les uns des autres, H ou OH, ou bien R(6) et R(7) ou, respectivement, R(8) et R(9) représentent ensemble l'atome d'oxygène d'un groupe carbonyle,

et au moins l'un des deux restes d'acides biliaires G1 et G2 étant raccourci d'un ou plusieurs atomes de carbone dans la chaîne latérale, c'est-à-dire que Z représente une liaison,

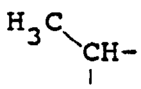 ou 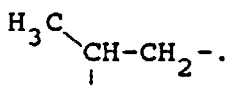

2. Médicament contenant un dérivé d'acide biliaire selon la revendication 1.

3. Médicament selon la revendication 2, destiné au traitement de l'hyperlipidémie.

4. Utilisation d'un dérivé d'acide biliaire selon la revendication 1, pour la fabrication d'un médicament destiné au traitement de l'hyperlipidémie.